# EUROPEAN PATENT APPLICATION

(11) **EP 4 353 814 A1**
(43) Date of publication of application: **17.04.2024**
(21) Application number: 22804720.5
(22) Date of filing: 18.05.2022
(51) Int. Cl.: C12N 1/21, C12N 1/15, C12N 1/19, C12N 5/10, C12N 15/31, C12N 15/54, C12N 15/60, C12P 13/00

(54) **RECOMBINANT MICROORGANISM HAVING DIAMINE PRODUCING ABILITY AND METHOD FOR MANUFACTURING DIAMINE**

(30) Priority: 19.05.2021 JP 2021084283; 20.05.2021 JP 2021085207
(71) Applicant: Asahi Kasei Kabushiki Kaisha, Tokyo 1000006 (JP)
(72) Inventor: MIYATAKE Ryo, Tokyo 100-0006 (JP); USHIKI Norisuke, Tokyo 100-0006 (JP); ISAKA Koji, Tokyo 100-0006 (JP)
(74) Representative: D Young & Co LLP
(86) International application number: PCT/JP2022/020689
(87) International publication number: WO 2022/244809

(57) **Abstract**

[Problem of invention] To provide a recombinant microorganism and a method for manufacturing a diamine that realize at least one of a simplified process and a reduction in effluent treatment cost in production of a diamine, and to provide a halophilic and/or alkalophilic recombinant microorganism having suppressed N-acetyldiamine production.

[Solution of problem] A halophilic and/or alkalophilic recombinant microorganism having a diamine producing ability, in which the diamine is represented by formula NH₂CH₂(CH₂)ₙCH₂NH₂ (in the formula, n is an integer of 0 to 10), and the recombinant microorganism resulting from modification of a halophilic and/or alkalophilic host microorganism so as to impart a diamine producing ability; and a halophilic and/or alkalophilic recombinant microorganism having a diamine producing ability, the recombinant microorganism containing one or more genetic modifications that suppress an N-acetylation enzyme that N-acetylates a diamine compound to produce an N-acetyldiamine compound.

## Description

### Technical Field

The present invention relates to a halophilic and/or alkalophilic recombinant microorganism having a diamine producing ability which is an industrially useful compound, and a method for manufacturing a diamine using the recombinant microorganism. The present invention also relates to a halophilic and/or alkalophilic recombinant microorganism having suppressed N-acetyldiamine production.

### Background Art

Due to disasters or climate change accompanying global warming in recent years, there is a strong demand for the development of a sustainable process aiming at symbiosis with the global environment and environmental conservation. Among them, a bioprocess, which is a substance production process in which a renewable raw material can be used and a biological reaction is utilized, has been greatly expected. So far, fermentative production processes for various chemical products have been developed. For example, a fermentative production process of polyols used for a polyurethane raw material, a polyester raw material, a plasticizer raw material, a pharmaceutical intermediate, and the like has been proposed.

A diamine, together with a dicarboxylic acid such as adipic acid, is an industrially important compound as a raw material of a polyamide (PA66 (6,6-nylon) or the like).

For example, 1,6-hexamethylenediamine (also called 1,6-diaminohexane or hexamethylenediamine, a compound having a molecular formula C₆H₁₂N₂) is widely used in fiber and resin applications as a raw material of nylon 66 (PA66) and is expected to be in demand worldwide. In addition, hexamethylenediamine is used as a urethane raw material, an agricultural chemical, and an intermediate of a pharmaceutical via isocyanate. Hexamethylenediamine is synthesized by obtaining adiponitrile through hydrocyanation of butadiene, electrolytic dimerization of acrylonitrile, or nitrilation of adipic acid, and performing hydrogenation using nickel or the like as a catalyst (Non Patent Literature 1). Hexamethylenediamine is industrially produced by the method, but adiponitrile is once synthesized, and then, a hydrogenation reaction is performed.

As a method for manufacturing hexamethylenediamine using microorganisms, methods for producing a diamine from an intracellular dicarboxylic acid, an aminocarboxylic acid, and a dialdehyde by combining an exogeneous enzyme, for example, a carboxylic acid decarboxylase and an aminotransferase (Patent Literature 1 and Patent Literature 2) have been reported. In Patent Literature 1, based on a metabolic simulation in in silico, prediction was made and examples were provided regarding an enzyme gene whose yield is expected to be improved due to deletion and/or disruption in a host microorganism modified to have a hexamethylenediamine production pathway. However, a by-product derived from an intermediate in the hexamethylenediamine production pathway and a method for suppressing the same are not mentioned at all. Patent Literature 2 describes a method for producing hexamethylenediamine by an enzymatic reaction pathway via 6-hydroxyhexanoic acid. However, a method for producing a by-product derived from an intermediate in a hexamethylenediamine production pathway newly constructed by a genetic modification, or a method for suppressing the same is not mentioned at all.

1,5-Pentamethylenediamine (also called 1,5-diaminopentane or cadaverine, a compound having the molecular formula C₅H₁₄N₂) is expected to be in demand for fiber and resin applications as a raw material of nylon (PA56). PA56 fibers have strength and heat resistance equivalent to those of PA66, and exhibit high hygroscopicity and high moisture releasability, and thus are expected to be newly developed in the market. In addition, 1,5-pentamethylenediamine is also attracting attention as a urethane raw material, an agricultural chemical, and an intermediate of a pharmaceutical, via isocyanate.

Although an efficient chemical synthesis method using a petrochemical raw material of 1,5-pentamethylenediamine has not yet been established, it is known to be easily produced by enzymatic decarboxylation of L-lysine in biosynthesis, and PA56 resin formed using biomass-derived 1,5-pentamethylenediamine as a raw material is industrially attracting attention as one of bioplastics from the viewpoint of reducing an environmental load (Non Patent Literature 2) .

In addition, a fermentative production process for diamines from renewable raw materials has also been proposed. Examples of an amine compound that can be produced by fermentation include 1,5-pentamethylenediamine and hexamethylenediamine, and these compounds can be used as raw materials for polymer production in the manufacture of chemical products (Patent Literatures 3 to 7 and Non Patent Literature 3).

As a method for manufacturing 1,5-pentamethylenediamine by a microorganism, there is a known method in which Escherichia coli highly expressing lysine decarboxylase is cultured, lysine as a precursor is reacted with the enzyme to decarboxylate thereby obtaining 1,5-pentamethylenediamine (Patent Literature 8). In addition, there has also been proposed a method for producing 1,5-pentamethylenediamine from glucose by culturing coryneform bacteria in which an activity of a lysine decarboxylase gene is increased and an activity of a gene that plays an important role in lysine biosynthesis is reduced (Patent Literature 9).

On the other hand, as a method for separating and purifying a diamine from a culture solution, for example, a method in which 1,5-pentamethylenediamine is released by adding an alkali solution such as sodium hydroxide and then extraction is performed by using an appropriate solvent is known (Patent Literatures 10, 11, and 12). In addition, a method in which a 1,5-pentamethylenediamine carbonate aqueous solution is thermally decomposed to be separated into crude 1,5-pentamethylenediamine and carbon dioxide and then a diamine is distilled and purified has also been proposed (Patent Literature 13). Furthermore, in the fermentative production of diamines by microorganisms, it has been proposed to separate (dissociate) a free base of a diamine and carbon dioxide from diamine carbonates and/or diamine carbamates neutralized with carbon dioxide which is added from the outside or generated by metabolism, and then extract the diamine with an organic solvent (Patent Literature 14).

However, the method proposed so far has the following disadvantages. First, regarding the fermentative production of diamines by microorganisms, for example, when lysine is converted to 1,5-pentamethylenediamine, a pH in a medium increases, but Escherichia coli and coryneform bacteria used in the methods described in Patent Literatures 8 and 9 can generally grow only around at a pH of 7 to 8, and the growth is significantly inhibited in an environment with a pH of 9 or higher. Therefore, when such a microorganism is used as a catalyst, it is required to control the pH to a range in which the growth of the microorganism is not inhibited by appropriately adding an acid or an alkali solution, and the production process becomes complicated.

Next, regarding the diamine purification step, in the methods described in Patent Literatures 11 and 14, a polar organic solvent such as chloroform or hexane is used for extraction, but the organic solvent is often harmful, and its handling is not preferable. In addition, the low extraction efficiency greatly affects the production cost. Furthermore, when the organic solvent to be used is recovered in order to reduce the production cost, the production process becomes complicated. In addition, in the method described in Patent Literature 12, purification is performed by a crystallization method, but a crystallization rate is 40 to 45%, and a high yield cannot be expected.

In the method described in Patent Literature 10, a large amount of salt is generated as a by-product in the diamine releasing step. For example, in a case where 50 g/L of cadaverine is produced by performing culture in which neutrality is maintained while adding sulfuric acid, sodium sulfate of about 100 g/L at maximum is generated as a by-product due to the presence of sulfate ions which are counter ions of cadaverine. It is likely to be significantly costly to dispose of a waste liquid containing the by-product. Furthermore, in the method described in Patent Literature 14, handling of an aqueous phase containing inorganic salts after extraction with a diamine solvent is not mentioned, but this method may also cost a lot for a waste liquid treatment.

Thus, there is room for further improvement in existing diamine production.

### Citation List

### Patent Literature

Patent Literature 1: JP 2015-146810 A
Patent Literature 2: JP 2017-544854 A
Patent Literature 3: JP 2012-188407 A
Patent Literature 4: JP 2012-525856 A
Patent Literature 5: JP 2016-538870 A
Patent Literature 6: JP 2016-501031 A
Patent Literature 7: JP 2017-533734 A
Patent Literature 8: JP 5553394 B2
Patent Literature 9: JP 5210295 B2
Patent Literature 10: JP 5646345 B2
Patent Literature 11: JP 4196620 B2
Patent Literature 12: JP 2005-6650 A
Patent Literature 13: JP 5930594 B2
Patent Literature 14: JP 2018-500911 A

### Non Patent Literature

Non Patent Literature 1: Process Economics Program Report 31B (IHS market)
Non Patent Literature 2: Tsuge, Y. et al., Engineering cell factories for producing building block chemicals for bio-polymer synthesis., Microb.Cell Fact., Vol., 15, 19 (2016)
Non Patent Literature 3: A Novel Process For Cadaverine Bio-Production Using a Consorsium of Two Engineering Escherichia Coli, Wang j Et Al. Frontiers in Microbiology (2018)

### Summary of Invention

### Technical Problem

In view of the above current situation, an object of the present invention is to provide a recombinant microorganism and a method for manufacturing a diamine that can realize at least one of a simplified process and a reduction in wastewater treatment cost in production of a diamine.

Another object of the present invention is to provide a halophilic and/or alkalophilic recombinant microorganism having suppressed N-acetyldiamine production.

### Solution to Problem

In order to solve the above problems, as a result of intensive studies, the present inventors have found that at least one of a simplified process and a reduction in wastewater treatment cost can be realized in production of a diamine by the recombinant microorganism of the present invention being obtained by modifying a host microorganism having at least one of halophilicity and alkalinity so as to have a diamine producing ability.

That is, the present invention provides the following:
[1] A halophilic and/or alkalophilic recombinant microorganism having a diamine producing ability,
   in which the diamine is represented by formula NH₂CH₂(CH₂)ₙCH₂NH₂ (in the formula, n is an integer of 0 to 10), and
   the recombinant microorganism resulting from modification of a halophilic and/or alkalophilic host microorganism so as to impart a diamine producing ability;
[2] The recombinant microorganism according to [1], in which n in the formula is 3 or 4;
[3] The recombinant microorganism according to [2], in which the host microorganism includes one or more genetic manipulations to induce overproduction of L-lysine decarboxylase (EC 4.1.1.18);
[4] The recombinant microorganism according to [3], in which the genetic manipulation is one or more manipulations selected from the group consisting of the following (A), (B), (C), (D), and (E):
   (A) a manipulation of introducing an exogenous gene encoding the L-lysine decarboxylase into the host microorganism;
   (B) a manipulation of increasing a copy number of an endogenous gene of the L-lysine decarboxylase in the host microorganism;
   (C) a manipulation of introducing a mutation into an expression regulatory region of the endogenous gene of the L-lysine decarboxylase in the host microorganism;
   (D) a manipulation of replacing the expression regulatory region of the endogenous gene of the L-lysine decarboxylase in the host microorganism by an exogenous regulatory region which enables high expression; and
   (E) a manipulation of deleting the regulatory region of the endogenous gene of the L-lysine decarboxylase in the host microorganism;
[5] The recombinant microorganism according to any one of [2] to [4], in which the recombinant microorganism contains:
   a base sequence having a homology of 85% or more, 90% or more, 92% or more, 95% or more, 98% or more, or 99% or more with a base sequence set forth in SEQ ID NO: 2 or SEQ ID NO: 4, or a base sequence having a homology of 85% or more, 90% or more, 92% or more, 95% or more, 98% or more, or 99% or more with a base sequence encoding an amino acid sequence set forth in SEQ ID NO: 1 or 3;
[6] The recombinant microorganism according to any one of [2] to [5], in which the recombinant microorganism is further modified by a mutation manipulation or a genetic recombination manipulation for improving a lysine producing ability;
[7] The recombinant microorganism according to [6], in which the mutation manipulation or the genetic recombination manipulation is a manipulation of canceling a feedback inhibition on at least one of aspartokinase III (EC 2.7.2.4) and 4-hydroxy-tetrahydrodipicolinate synthase (EC 4.3.3.7);
[8] The recombinant microorganism according to any one of [1] to [7], in which the host microorganism is selected from the group consisting of Bacillus pseudofirmus, Bacillus halodurans, and Bacillus marmarensis;
[9] The recombinant microorganism according to any one of [1] to [8], in which the host microorganism is Bacillus pseudofirmus;
[10] A method for manufacturing a diamine represented by formula NH₂CH₂(CH₂)ₙCH₂NH₂ (in the formula, n is an integer of 0 to 10) using the recombinant microorganism according to any one of [1] to [9];
[11] The manufacturing method according to [10], in which n in the formula is 3 or 4;
[12] The manufacturing method according to [10] or [11], the method including a culture step of culturing the recombinant microorganism in a medium containing 5 mass% or more of an inorganic salt to obtain a culture solution containing a diamine;
[13] The manufacturing method according to any one of [10] to [12], the method including:
   a culture step of culturing the recombinant microorganism to obtain a culture solution containing bacterial cells; and
   a reaction step of bringing the culture solution and/or the bacterial cells into contact with an aqueous solution containing 5 mass% or more of an inorganic salt and lysine to obtain a reaction solution containing 1,5-pentanediamine;
[14] The manufacturing method according to [12] or [13], the method including a removal step of removing the recombinant microorganism from the culture solution or the reaction solution;
[15] The manufacturing method according to [14], the method including a concentration step of concentrating the culture solution or the reaction solution;
[16] The manufacturing method according to [15], the method including a pH adjustment step of concentrating the culture solution or the reaction solution by the concentration step and then adjusting a pH of the concentrated culture solution or reaction solution to 12 or higher;
[17] The manufacturing method according to any one of [12] to [16], the method including a separation step of performing a phase separation into a phase containing a diamine and an aqueous phase containing the inorganic salt from the culture solution or the reaction solution;
[18] The manufacturing method according to any one of [12] to [17], in which in the separation step, a phase separation into a phase containing a diamine and an organic solvent and an aqueous phase is performed by adding an organic solvent to the culture solution or the reaction solution;
[19] The manufacturing method according to [17] or [18], in which in the separation step, an alkali compound is not added;
[20] The manufacturing method according to any one of [12] to [19], in which the inorganic salt is sodium carbonate and/or sodium sulfate;
[21] The manufacturing method according to any one of [12] to [20], in which
   the diamine produced by the culture step and/or the reaction step is in one or more forms selected from the group consisting of a carbonate salt, a bicarbonate salt, a bisbicarbonate salt, a carbamate salt, and a biscarbamate salt, and
   in the concentration step, the diamine in the forms of the salts is converted into a free base and carbon dioxide to separate the carbon dioxide;
[22] The manufacturing method according to any one of [18] to [21], in which the organic solvent is one or more selected from the group consisting of hexane, butanol, and 2-ethyl-1-hexanol;
[23] The manufacturing method according to any one of [12] to [22], in which the diamine is produced by fermentation of at least one or more of sugar, carbon dioxide, synthetic gas, methanol, and amino acid by the recombinant microorganism;
[24] The manufacturing method according to any one of [17] to [23], the method including reusing the separated aqueous phase containing the inorganic salt as a culture solution; and
[25] The manufacturing method according to any one of [12] to [24], the method including a purification step of purifying the obtained diamine.

Furthermore, the present inventors have conducted intensive studies in order to solve the above further problems. Specifically, as described above, the present inventors have found that the use of a halophilic and/or alkalophilic recombinant microorganism in order to simplify the diamine production process and reduce the wastewater treatment cost, and have found that the use of the halophilic and/or alkalophilic recombinant microorganism as a host microorganism has made it possible to simplify a pH adjustment step during production of 1,5-pentamethylenediamine and to reuse, as a medium, high-concentration salt-containing water generated in a 1,5-pentamethylenediamine releasing step, but in the production process of 1,5-pentamethylenediamine by the halophilic and/or alkalophilic recombinant microorganism, N-acetylcadaverine in which an N-terminal amino group of 1,5-pentamethylenediamine is acetylated is produced as a by-product.

N-acetylation of 1,5-pentamethylenediamine is catalyzed by an acetyltransferase that is endogenous to microbial cells. In Corynebacterium coryneum, an N-acetylation enzyme for 1,5-pentamethylenediamine is identified, and N-acetylation of 1,5-pentamethylenediamine is suppressed by disrupting a gene encoding the enzyme (Patent Literature 9 and JP 5960604 B2).

However, as a result of performing homology search on genome information of a halophilic and/or alkalophilic recombinant microorganism, it has been found that homologues of the genes do not exist. Therefore, in the halophilic and/or alkalophilic recombinant microorganism, it was predicted that any acetyltransferase, which is phylogenetically different from Corynebacterium coryneum, catalyzes the acetylation of 1,5-pentamethylenediamine.

Therefore, in order to suppress the acetylation of the diamine in the production of 1,5-pentamethylenediamine by the halophilic and/or alkalophilic recombinant microorganism, it is first necessary to identify an enzyme that catalyzes the acetylation of 1,5-pentamethylenediamine.

As a result of the intensive studies described above, the present inventors have succeeded in identifying a gene encoding an enzyme that catalyzes acetylation of 1,5-pentamethylenediamine from a genome sequence of a halophilic and/or alkalophilic host microorganism, and have modified the gene, thereby suppressing production of N-acetyldiamine as a by-product in the halophilic and/or alkalophilic host microorganism.

That is, the present invention further provides the followings:
[26] A halophilic and/or alkalophilic recombinant microorganism having a diamine producing ability, the recombinant microorganism including one or more genetic modifications that suppress an N-acetylation enzyme that N-acetylates a diamine compound to produce an N-acetyldiamine compound;
[27] The recombinant microorganism according to [26], in which the genetic modification is:
   · a modification that suppresses expression of an endogenous gene encoding the N-acetylation enzyme; or
   · a modification that reduces an activity of the N-acetylation enzyme;
[28] The recombinant microorganism according to [26] or [27], in which an N-acetyldiamine compound producing ability is suppressed or eliminated as compared with the producing ability of a non-mutant strain that does not contain the genetic modification;
[29] The recombinant microorganism according to any one of [26] to [28], in which a gene encoding the N-acetylation enzyme is yjbC gene;
[30] The recombinant microorganism according to any one of [26] to [29], in which
   the N-acetylation enzyme:
   (A) (A-1) consists of an amino acid sequence set forth in SEQ ID NO: 23;
   (A-2) consists of an amino acid sequence having a sequence identity of 85% or more, 90% or more, 95% or more, 97% or more, 98% or more, or 99% or more with the amino acid sequence set forth in SEQ ID NO: 23, and has an enzymatic activity to N-acetylate a diamine compound to produce an N-acetyldiamine compound; or
   (A-3) consists of an amino acid sequence in which 1 to 10, 1 to 7, 1 to 5, or 1 to 3 amino acids are deleted, substituted, inserted, and/or added in the amino acid sequence set forth in SEQ ID NO: 23, and has an enzymatic activity to N-acetylate a diamine compound to produce an N-acetyldiamine compound, or
   the N-acetylation enzyme is encoded by:
      (B) (B-1) DNA that consists of a base sequence set forth in SEQ ID NO: 24;
      (B-2) DNA that hybridizes with DNA containing a base sequence complementary to the base sequence set forth in SEQ ID NO: 24 under stringent conditions, and encodes a protein having an enzymatic activity that N-acetylates a diamine compound to produce an N-acetyldiamine compound;
      (B-3) DNA that consists of a base sequence having a sequence identity of 85% or more, 90% or more, 95% or more, 97% or more, 98% or more, or 99% or more with the base sequence set forth in SEQ ID NO: 24, and encodes a protein having an enzymatic activity that N-acetylates a diamine compound to produce an N-acetyldiamine compound;
      (B-4) DNA that encodes a protein consisting of an amino acid sequence in which 1 to 10, 1 to 7, 1 to 5, or 1 to 3 amino acids are deleted, substituted, inserted, and/or added in an amino acid sequence of a protein encoded by the base sequence set forth in SEQ ID NO: 24, and encodes a protein having an enzymatic activity that N-acetylates a diamine compound to produce an N-acetyldiamine compound; or
      (B-5) DNA that consists of a degenerate isomer of the base sequence set forth in SEQ ID NO: 24;
[31] The recombinant microorganism according to any one of [26] to [30], the recombinant microorganism resulting from one or more genetic modifications of a halophilic and/or alkalophilic host microorganism to suppress the N-acetylation enzyme;
[32] The recombinant microorganism according to any one of [26] to [31], in which the host microorganism is selected from the group consisting of Bacillus pseudofirmus, Bacillus halodurans, and Bacillus marmarensis;
[33] The recombinant microorganism according to any one of [26] to [32], in which the diamine compound is represented by formula NH₂CH₂(CH₂)ₙCH₂NH₂ (in the formula, n is 0, 1, 2, 3, 4, 5, 6, 7, 8, 9, or 10).
[34] The recombinant microorganism according to any one of [26] to [33], in which the diamine compound is cadaverine;
[35] A method for manufacturing a diamine compound, the method including a culture step of culturing the recombinant microorganism according to any one of [26] to [34] to obtain a culture of the recombinant microorganism and/or an extract of the culture;
[36] The manufacturing method according to [35], the method further including a mixing step of mixing the culture and/or the extract of the culture with a substrate compound to obtain a mixed solution; and
[37] The manufacturing method according to [35] or [36], the method further including a recovery step of recovering a diamine compound from the culture or the mixed solution.

### Effects of Invention

According to the present invention, at least one of the simplified process and the reduction in wastewater treatment cost can be realized in the production of the diamine.

In addition, according to the present invention, in the production of a diamine using a halophilic and/or alkalophilic diamine-producing bacterium, the production of N-acetyldiamine as a by-product can be suppressed.

### Brief Description of Drawings

Fig. 1 is a diagram illustrating an example of a hexamethylenediamine production pathway.
Fig. 2A is a diagram illustrating an amino acid sequence of each enzyme.
Fig. 2B is a diagram illustrating an amino acid sequence of each enzyme.
Fig. 3 is a diagram illustrating an amino acid sequence (SEQ ID NO: 23) of yjbC enzyme of Bacillus psuedofirmus.
Fig. 4 is a diagram illustrating a base sequence (SEQ ID NO: 24) of yjbC gene of Bacillus psuedofirmus.
Fig. 5A is a diagram illustrating a base sequence of a primer.
Fig. 5B is a diagram illustrating a base sequence of a primer.

### Description of Embodiments

Hereinafter, modes for carrying out the present invention will be described in detail. Note that the present invention is not limited to the following embodiments, and various modifications can be made within the scope of the gist of the present invention. In addition, unless otherwise specified, genetic manipulations such as acquisition of DNA and preparation of a vector and transformation described in the present specification can be performed by the methods described in known documents such as Molecular Cloning 4th Edition (Cold Spring Harbor Laboratory Press, 2012), Current Protocols in Molecular Biology (Greene Publishing Associates and Wiley-Interscience), and genetic engineering experimental note (Takaaki Tamura, YODOSHA CO., LTD.). In the present specification, unless otherwise specified, nucleotide sequences are described from the 5' direction to the 3' direction. In the present specification, the terms "polypeptide" and "protein" are used interchangeably. In addition, the "genetically modified microorganism" is simply referred to as a "recombinant microorganism".

In the present specification, a range of numerical values indicated by "to" represents a range including numerical values described before and after "to" as the minimum value and the maximum value, respectively. In the range of the numerical values described in stages in the present specification, an upper limit value or a lower limit value of a range of numerical values of a certain stage can be arbitrarily combined with an upper limit value or a lower limit value of a range of numerical values of another stage.

In the present specification, the term "endogenous" or "endogenous property" is used to mean that the host microorganism that is not modified by a genetic modification has the gene referred to or the protein encoded by the same (typically, an enzyme), regardless of whether the gene is functionally expressed to the extent that a predominant biochemical reaction can proceed in the host cell. The terms "internal property" and "endogenous property" are used interchangeably in the present specification.

In the present specification, the term "exogenous" or "exogenous property" is used to mean that a gene or a nucleic acid sequence is introduced into a host in a case where a pre-genetically modified host microorganism does not have a gene to be introduced, in a case where an enzyme is not substantially expressed by the gene, or in a case where an amino acid sequence of the enzyme is encoded by a different gene but does not express a corresponding endogenous enzymatic activity after the genetic modification. The terms "external property" and "exogenous property" are used interchangeably in the present specification.

In the present specification, regarding a microorganism, the phrase "having a diamine producing ability" refers to a microorganism that produces a diamine in any stage of a diamine-producing process using the microorganism. Specifically, a diamine may be contained in a culture solution obtained by culturing the microorganism, or a diamine may be produced by adding a diamine precursor, for example, a carboxylic acid compound, an aldehyde compound, and/or a carbonyl compound to a medium and culturing a microorganism that converts the diamine precursor into a diamine. A "microorganism having a diamine producing ability" includes a microorganism having one or more of these properties.

Furthermore, regarding a recombinant microorganism, the phrase "having a diamine producing ability" means that the microorganism has a diamine production pathway. In the present specification, regarding a compound, the microorganism "having a production pathway" means that the microorganism can express a sufficient amount of an enzyme to allow each reaction step of the production pathway of the compound to proceed, and can biosynthesize the compound. The recombinant microorganism of the present invention may be obtained using a host microorganism that originally has an ability to produce a diamine, or may be obtained by modifying a halophilic and/or alkalophilic host microorganism that originally does not have an ability to produce a diamine so that the host microorganism has a diamine producing ability.

In relation to the present invention, a diamine compound is represented by the formula NH₂CH₂(CH₂)ₙCH₂NH₂. In the formula, n is, for example, 0, 1, 2, 3, 4, 5, 6, 7, 8, 9, or 10, preferably 2, 3, 4, or 5, more preferably 2, 3, or 4, and particularly preferably 3 or 4.

Examples of the diamine compound include ethylenediamine, propylenediamine, tetramethylenediamine (for example, putrescine), pentamethylenediamine (for example, cadaverine), hexamethylenediamine, and heptamethylenediamine. In a preferred aspect, the diamine compound is hexamethylenediamine or cadaverine.

### <1> Invention A: Recombinant Microorganism having Diamine Producing Ability and Method for Manufacturing Diamine

The recombinant microorganism according to the present invention is a microorganism having a diamine producing ability, and specifically, is obtained by modifying a host microorganism having at least one of halophilicity and alkalinity so as to impart a diamine producing ability.

In natural world, there are microorganisms having alkaliphilic property capable of growing in an alkaline environment with a pH of 9 or higher, halophilic property capable of growing in an environment with a NaCl concentration of 0.2 M or higher, or both of these properties. The present inventors have considered that when the alkalophilic microorganisms can be used for production of a diamine, the microorganisms can grow even when the pH increases due to an increase in a product concentration in an enzyme conversion step or a culture step, and thus it is possible to omit pH adjustment with addition of an acid solution. In addition, it was considered that when the halophilic microorganisms can be used for the production of a diamine, high-concentration salt-containing water generated in a releasing step can be reused as a medium. Note that an example in which a microorganism having at least one of halophilicity and alkalinity is applied to diamine production has not been reported so far.

The present inventors used a microorganism having at least one of halophilicity and alkalinity as a host microorganism and attempted to modify the microorganism so as to impart a diamine producing ability, and have found that according to the obtained recombinant microorganism, at least one of the simplified process and the reduction in wastewater treatment cost was realized.

That is, the present inventors have succeeded in imparting a diamine producing ability to a halophilic and/or alkalophilic microorganism, and have found that when such a microorganism is used, even in a case where the pH increases due to an increase in the product concentration in an enzyme conversion step, a culture step, or the like, the microorganism can grow without adjusting the pH such as addition of an acid solution, and in a more preferred aspect, high concentration salt-containing water generated when a diamine is isolated can be reused as a medium.

In the manufacture of the recombinant microorganism of the embodiment of the present invention, for example, a technique of introducing an exogenous gene into cells of a host microorganism having at least one of halophilicity and alkalinity, a technique of introducing any exogenous gene sequence into a genome sequence, or a technique of removing an unnecessary gene sequence from a genome sequence can be used.

That is, the following techniques can be exemplified:
(1) a technique of transforming a host microorganism having halophilicity, alkalophilicity, or both properties by combining a promoter that can be expressed in the host cell and a gene involved in diamine production;
(2) the technique of (1) above, in which a plasmid vector that is stably replicated in a cell of a microorganism having halophilicity, alkalophilicity, or both properties;
(2') the technique of (2) above, in which the plasmid vector targeted by the technique is pUB110;
(3) the technique of (1) above, characterized in that a desired trait is stably expressed in a cell of a microorganism having halophilicity, alkalophilicity, or both properties;
(4) a technique of arbitrarily modifying a gene sequence on a chromosome by combining a temperature-sensitive plasmid vector and negative selection;
(5) the technique of (4) above, characterized in that replication of a plasmid of the microorganism is stopped at a temperature of 37°C or higher or the copy number of the plasmids of the microorganism is reduced;
(6) the technique of (4) above, characterized in that selection (negative selection) of microorganisms that do not have a specific gene is performed by inhibiting growth of microorganisms that have a specific gene in the presence of substances such as sucrose and 4-chlorophenylalanine;
(6') the technique of (6) above, characterized in that a specific gene targeted by the technique is a levansucrase gene (sacB gene); and
(6") the technique of (6) above, characterized in that a specific gene targeted by the technique is a host-derived phenylalanine tRNA synthetase α-subunit gene (pheS gene) in which a mutation is introduced into a base sequence.

In a preferred aspect of the present invention, a high expression promoter and an exogenous gene are retained in a plasmid vector, and the plasmid vector is introduced into a cell of a microorganism having halophilicity, alkalophilicity, or both properties, such that the exogenous gene is stably highly expressed. In addition, in another preferred aspect, a genome sequence retained by a microorganism having halophilicity, alkalophilicity, or both properties is arbitrarily edited by a technique in which a temperature-sensitive plasmid vector and negative selection are combined. A cell strain in which a gene is introduced or a genome sequence is edited using the technique may be a wild-type strain in a usual sense, or may be an auxotrophic mutant strain or an antibiotic resistant mutant strain derived from the wild-type strain. Furthermore, the cell strain that can be used as the host cell of the present invention may be already subjected to transformation so as to have various marker genes related to mutation as described above. These techniques can provide properties beneficial to the production, maintenance, and/or management of the recombinant microorganism of the present invention.

In relation to the present invention, an alkalophilic microorganism that can be used as a host microorganism is a kind of an extreme environmental microorganism exhibiting various distributions, and is a generic term for microorganisms that can grow even in an environment with a pH of 9 or higher. These microorganisms are classified into absolutely alkalophilic microorganisms capable of growing only in an environment with a pH of 9 or higher and facultative alkalophilic microorganisms capable of growing even at a pH of lower than 9 although having an optimum growth at a pH of 9 or higher. Some of them can also grow in a strongly alkaline environment with a pH of 12 or higher. All of these are the alkalophilic microorganisms of the present invention.

In relation to the present invention, the halophilic microorganism that can be used as a host microorganism is a generic term for microorganisms that can respond to high-concentration salt stress. In the classification of bacteria based on an optimal growth salt concentration, these bacteria are classified into non-halophilic bacteria in which an optimal growth salt concentration of sodium chloride is 0 to 0.2 M, low-halophilic bacteria in which an optimal growth salt concentration of sodium chloride is 0.2 to 0.5 M, medium-halophilic bacteria in which an optimal growth salt concentration of sodium chloride is 0.5 to 2.5 M, and highly halophilic bacteria in which an optimal growth salt concentration of sodium chloride is 2.5 to 5.2 M. All of these bacteria except the non-halophilic bacteria are the halophilic microorganisms of the present invention.

Examples of the microorganism having halophilicity, alkalophilicity, or both properties that can be used as the host microorganism of the present invention include various microorganisms, and non-limiting examples thereof include bacteria of the genus Bacillus, the genus Halomonas, the genus Halobacteroides, the genus Salinibacter, the genus Alkaliphilus, the genus Clostridium, and the genus Anaerobranca. The host microorganism of the present invention is preferably a bacterium of the genus Bacillus. Among the microorganisms of the genus Bacillus, Bacillus pseudofirmus, Bacillus halodurans, and Bacillus marmarensis are preferable, and Bacillus psuedofirmus is more preferable.

In a preferred aspect of the present invention, modifications for constructing a new diamine biosynthesis pathway are performed in the host microorganism. For example, when the diamine is 1,5-pentamethylenediamine, the recombinant microorganism is obtained by introducing one or more L-lysine decarboxylase (EC 4.1.1.18) enzyme genes into cells of the host microorganism in order to construct a new 1,5-pentamethylenediamine biosynthesis pathway. Here, L-lysine decarboxylase (EC 4.1.1.18) is an enzyme that catalyzes a reaction of decarboxylating L-lysine to produce 1,5-pentanediamine. Alternatively, the recombinant microorganism is obtained by inserting the enzyme gene sequence into the genome sequence of the host microorganism.

Such a manipulation induces overproduction of L-lysine decarboxylase (EC 4.1.1.18) in the obtained recombinant microorganism. That is, the recombinant microorganism according to the present invention is modified by one or more genetic manipulations to induce overproduction of L-lysine decarboxylase (EC 4.1.1.18).

In a case where the diamine is 1,5-pentamethylenediamine, the genetic manipulation may be, for example, one or more genetic manipulations selected from the group consisting of the following (A), (B), (C), (D), and (E):
(A) a manipulation of introducing an exogenous gene encoding the L-lysine decarboxylase into the host microorganism;
(B) a manipulation of increasing a copy number of an endogenous gene of the L-lysine decarboxylase in the host microorganism;
(C) a manipulation of introducing a mutation into an expression regulatory region of the endogenous gene of the L-lysine decarboxylase in the host microorganism;
(D) a manipulation of replacing the expression regulatory region of the endogenous gene of the L-lysine decarboxylase in the host microorganism by an exogenous regulatory region which enables high expression; and
(E) a manipulation of deleting the regulatory region of the endogenous gene of the L-lysine decarboxylase in the host microorganism.

Representative examples of the gene for L-lysine decarboxylase (EC 4.1.1.18) include cadA and ldcC of Escherichia coli. An amino acid sequence of Escherichia coli cadA enzyme is set forth in SEQ ID NO: 1, and a base sequence of Escherichia coli cadA is set forth in SEQ ID NO: 2. In addition, an amino acid sequence of Escherichia coli ldcC enzyme is set forth in SEQ ID NO: 3, and a base sequence of Escherichia coli ldcC is set forth in SEQ ID NO: 4.
[Chem. 1]
(SEQ ID NO: 1) 
MNVIAILNHMGVYFKEEPIRELHRALERLNFQIVYPNDRDDLLKLIENNARLCGVIFDWD
KYNLELCEEISKMNENLPLYAFANTYSTLDVSLNDLRLQISFFEYALGAAEDIANKIKQT
TDEYINTILPPLTKALFKYVREGKYTFCTPGHMGGTAFQKSPVGSLFYDFFGPNTMKSDI
SISVSELGSLLDHSGPHKEAEQYIARVFNADRSYMVTNGTSTANKIVGMYSAPAGSTILI
DRNCHKSLTHLMMMSDVTPIYFRPTRNAYGILGGIPQSEFQHATIAKRVKETPNATWPVH
AVITNSTYDGLLYNTDFIKKTLDVKSIHFDSAWVPYTNFSPIYEGKCGMSGGRVEGKVIY
ETQSTHKLLAAFSQASMIHVKGDVNEETFNEAYMMHTTTSPHYGIVASTETAAAMMKGNA
GKRLINGSIERAIKFRKEIKRLRTESDGWFFDVWQPDHIDTTECWPLRSDSTWHGFKNID
NEHMYLDPIKVTLLTPGMEKDGTMSDFGIPASIVAKYLDEHGIVVEKTGPYNLLFLFSIG
IDKTKALSLLRALTDFKRAFDLNLRVKNMLPSLYREDPEFYENMRIQELAQNIHKLIVHH
NLPDLMYRAFEVLPTMVMTPYAAFQKELHGMTEEVYLDEMVGRINANMILPYPPGVPLVM
PGEMITEESRPVLEFLQMLCEIGAHYPGFETDIHGAYRQADGRYTVKVLKEESKK
(SEQ ID NO: 2) 
atgaacgttattgcaatattgaatcacatgggggtttattttaaagaagaacccatccgt
gaacttcatcgcgcgcttgaacgtctgaacttccagattgtttacccgaacgaccgtgac
gacttattaaaactgatcgaaaacaatgcgcgtctgtgcggcgttatttttgactgggat
aaatataatctcgagctgtgcgaagaaattagcaaaatgaacgagaacctgccgttgtac
gcgttcgctaatacgtattccactctcgatgtaagcctgaatgacctgcgtttacagatt
agcttctttgaatatgcgctgggtgctgctgaagatattgctaataagatcaagcagacc
actgacgaatatatcaacactattctgcctccgctgactaaagcactgtttaaatatgtt
cgtgaaggtaaatatactttctgtactcctggtcacatgggcggtactgcattccagaaa
agcccggtaggtagcctgttctatgatttctttggtccgaataccatgaaatctgatatt
tccatttcagtatctgaactgggttctctgctggatcacagtggtccacacaaagaagca
gaacagtatatcgctcgcgtctttaacgcagaccgcagctacatggtgaccaacggtact
tccactgcgaacaaaattgttggtatgtactctgctccagcaggcagcaccattctgatt
gaccgtaactgccacaaatcgctgacccacctgatgatgatgagcgatgttacgccaatc
tatttccgcccgacccgtaacgcttacggtattcttggtggtatcccacagagtgaattc
cagcacgctaccattgctaagcgcgtgaaagaaacaccaaacgcaacctggccggtacat
gctgtaattaccaactctacctatgatggtctgctgtacaacaccgacttcatcaagaaa
acactggatgtgaaatccatccactttgactccgcgtgggtgccttacaccaacttctca
ccgatttacgaaggtaaatgcggtatgagcggtggccgtgtagaagggaaagtgatttac
gaaacccagtccactcacaaactgctggcggcgttctctcaggcttccatgatccacgtt
aaaggtgacgtaaacgaagaaacctttaacgaagcctacatgatgcacaccaccacttct
ccgcactacggtatcgtggcgtccactgaaaccgctgcggcgatgatgaaaggcaatgca
ggtaagcgtctgatcaacggttctattgaacgtgcgatcaaattccgtaaagagatcaaa
cgtctgagaacggaatctgatggctggttctttgatgtatggcagccggatcatatcgat
acgactgaatgctggccgctgcgttctgacagcacctggcacggcttcaaaaacatcgat
aacgagcacatgtatcttgacccgatcaaagtcaccctgctgactccggggatggaaaaa
gacggcaccatgagcgactttggtattccggccagcatcgtggcgaaatacctcgacgaa
catggcatcgttgttgagaaaaccggtccgtataacctgctgttcctgttcagcatcggt
atcgataagaccaaagcactgagcctgctgcgtgctctgactgactttaaacgtgcgttc
gacctgaacctgcgtgtgaaaaacatgctgccgtctctgtatcgtgaagatcctgaattc
tatgaaaacatgcgtattcaggaactggctcagaatatccacaaactgattgttcaccac
aatctgccggatctgatgtatcgcgcatttgaagtgctgccgacgatggtaatgactccg
tatgctgcattccagaaagagctgcacggtatgaccgaagaagtttacctcgacgaaatg
 
gtaggtcgtattaacgccaatatgatccttccgtacccgccgggagttcctctggtaatg
ccgggtgaaatgatcaccgaagaaagccgtccggttctggagttcctgcagatgctgtgt
gaaatcggcgctcactatccgggctttgaaaccgatattcacggtgcataccgtcaggct
gatggccgctataccgttaaggtattgaaagaagaaagcaaaaaataa
(SEQ ID NO: 3) 
MNIIAIMGPHGVFYKDEPIKELESALVAQGFQIIWPQNSVDLLKFIEHNPRICGVIFDWD
EYSLDLCSDINQLNEYLPLYAFINTHSTMDVSVQDMRMALWFFEYALGQAEDIAIRMRQY
TDEYLDNITPPFTKALFTYVKERKYTFCTPGHMGGTAYQKSPVGCLFYDFFGGNTLKADV
SISVTELGSLLDHTGPHLEAEEYIARTFGAEQSYIVTNGTSTSNKIVGMYAAPSGSTLLI
DRNCHKSLAHLLMMNDVVPVWLKPTRNALGILGGIPRREFTRDSIEEKVAATTQAQWPVH
AVITNSTYDGLLYNTDWIKQTLDVPSIHFDSAWVPYTHFHPIYQGKSGMSGERVAGKVIF
ETQSTHKMLAALSQASLIHIKGEYDEEAFNEAFMMHTTTSPSYPIVASVETAAAMLRGNP
GKRLINRSVERALHFRKEVQRLREESDGWFFDIWQPPQVDEAECWPVAPGEQWHGFNDAD
ADHMFLDPVKVTILTPGMDEQGNMSEEGIPAALVAKFLDERGIVVEKTGPYNLLFLFSIG
IDKTKAMGLLRGLTEFKRSYDLNLRIKNMLPDLYAEDPDFYRNMRIQDLAQGIHKLIRKH
DLPGLMLRAFDTLPEMIMTPHQAWQRQIKGEVETIALEQLVGRVSANMILPYPPGVPLLM
PGEMLTKESRTVLDFLLMLCSVGQHYPGFETDIHGAKQDEDGVYRVRVLKMAG
(SEQ ID NO: 4)

atgaacatcattgccattatgggaccgcatggcgtcttttataaagatgagcccatcaaa
gaactggagtcggcgctggtggcgcaaggctttcagattatctggccacaaaacagcgtt
gatttgctgaaatttatcgagcataaccctcgaatttgcggcgtgatttttgactgggat
gagtacagtctcgatttatgtagcgatatcaatcagcttaatgaatatctcccgctttat
gccttcatcaacacccactcgacgatggatgtcagcgtgcaggatatgcggatggcgctc
tggttttttgaatatgcgctggggcaggcggaagatatcgccattcgtatgcgtcagtac
accgacgaatatcttgataacattacaccgccgttcacgaaagccttgtttacctacgtc
aaagagcggaagtacaccttttgtacgccggggcatatgggcggcaccgcatatcaaaaa
agcccggttggctgtctgttttatgattttttcggcgggaatactcttaaggctgatgtc
tctatttcggtcaccgagcttggttcgttgctcgaccacaccgggccacacctggaagcg
gaagagtacatcgcgcggacttttggcgcggaacagagttatatcgttaccaacggaaca
tcgacgtcgaacaaaattgtgggtatgtacgccgcgccatccggcagtacgctgttgatc
gaccgcaattgtcataaatcgctggcgcatctgttgatgatgaacgatgtagtgccagtc
tggctgaaaccgacgcgtaatgcgttggggattcttggtgggatcccgcgccgtgaattt
actcgcgacagcatcgaagagaaagtcgctgctaccacgcaagcacaatggccggttcat
gcggtgatcaccaactccacctatgatggcttgctctacaacaccgactggatcaaacag
acgctggatgtcccgtcgattcacttcgattctgcctgggtgccgtacacccattttcat
ccgatctaccagggtaaaagtggtatgagcggcgagcgtgttgcgggaaaagtgatcttc
gaaacgcaatcgacccacaaaatgctggcggcgttatcgcaggcttcgctgatccacatt
aaaggcgagtatgacgaagaggcctttaacgaagcctttatgatgcataccaccacctcg
cccagttatcccattgttgcttcggttgagacggcggcggcgatgctgcgtggtaatccg
ggcaaacggctgattaaccgttcagtagaacgagctctgcattttcgcaaagaggtccag
cggctgcgggaagagtctgacggttggtttttcgatatctggcaaccgccgcaggtggat
gaagccgaatgctggcccgttgcgcctggcgaacagtggcacggctttaacgatgcggat
gccgatcatatgtttctcgatccggttaaagtcactattttgacaccggggatggacgag
cagggcaatatgagcgaggaggggatcccggcggcgctggtagcaaaattcctcgacgaa
cgtgggatcgtagtagagaaaaccggcccttataacctgctgtttctctttagtattggc

atggataaaacaaagcaatgggattatctgcgtggtgtgccggaatttaaacgctattac
gatctcaacctgcggatcaaaaatatgctacccgatctctatgcagaagatcccgatttc
taccgcaatatgcgtattcaggatctggcacaagggatccataagctgattcgtaaacac
gatcttcccggtttgatgttgcgggcattcgatactttgccggagatgatcatgacgcca
catcaggcatggcaacgacaaattaaaggcgaagtagaaaccattgcgctggaacaactg
gtcggtagagtatcggcaaatatgatcctgccttatccaccgggcgtaccgctgttgatg
cctggagaaatgctgaccaaagagagccgcacagtactcgattttctactgatgctttgt
tccgtcgggcaacattaccccggttttgaaacggatattcacggcgcgaaacaggacgaa
gacggcgtttaccgcgtacgagtcctaaaaatggcgggataa

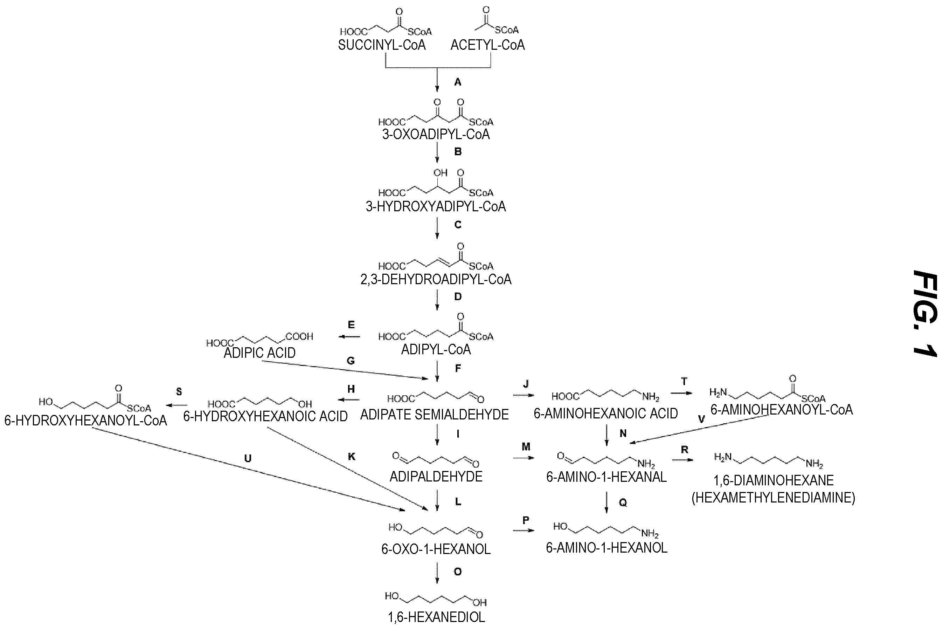

In a preferred aspect of the present invention, modifications for constructing a new diamine biosynthesis pathway have been performed in the host microorganism. For example, when the diamine is hexamethylenediamine, the recombinant microorganism is obtained by introducing one or more enzyme genes into cells of the host microorganism in order to construct a new hexamethylenediamine biosynthesis pathway. Alternatively, the recombinant microorganism is obtained by inserting the enzyme gene sequence into the genome sequence of the host microorganism. An example of the hexamethylenediamine biosynthesis pathway of the microorganism is illustrated in Fig. 1.

Hereinafter, the enzyme that catalyzes each reaction stage will be described.

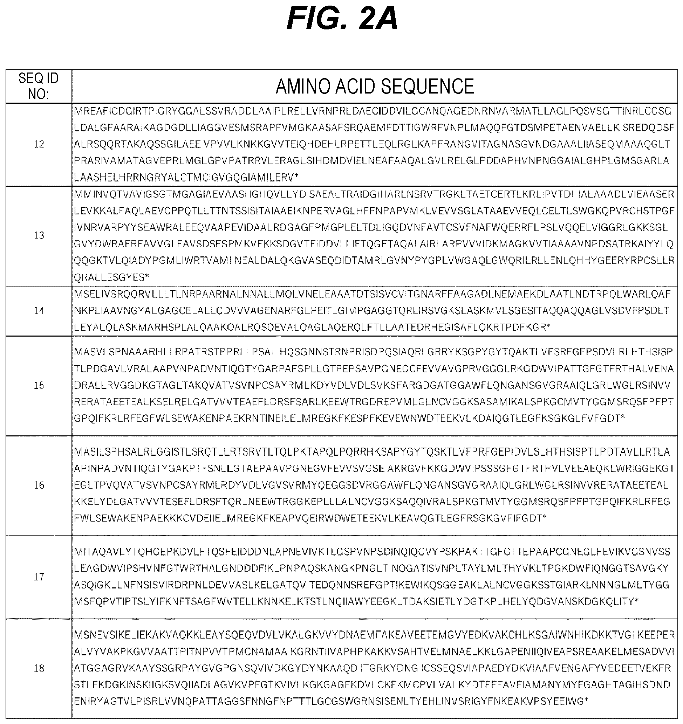

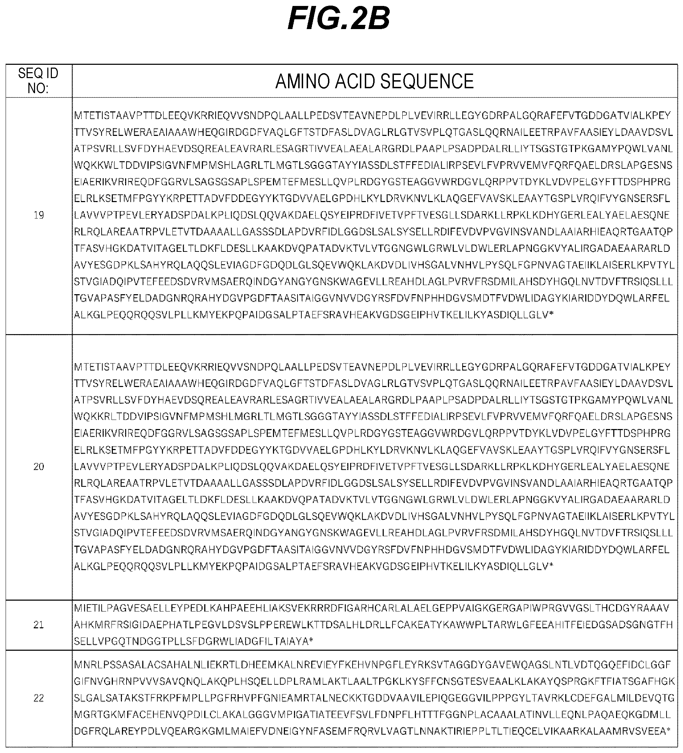

In the conversion of Step A in Fig. 1 (a succinyl-CoA:acetyl-CoA acyltransferase, or a 3-oxoadipyl-CoA thiolase), and succinyl-CoA and acetyl-CoA are condensed and converted into 3-oxoadipyl-CoA. Examples of the enzyme that can catalyze the present conversion include a β-ketothiolase. For example, enzymes classified into the group such as EC 2.3.1.9 (acetoacetyl-CoA thiolase), EC 2.3.1.16 (3-ketoacyl-CoA thiolase), or EC 2.3.1.174 (3-oxoadipyl-CoA thiolase) can be exemplified as enzymes that can have an activity for the present conversion. The enzyme used in the present invention is not limited as long as it has an activity for the present conversion, and for example, Escherichia coli-derived PaaJ consisting of an amino acid sequence set forth in SEQ ID NO: 12 is used (Fig. 2).

In the conversion of Step B in Fig. 1 (3-hydroxyadipyl-CoA dehydrogenase), 3-oxoadipyl-CoA is converted into 3-hydroxyadipyl-CoA. Examples of the enzyme that can catalyze the present conversion include oxidoreductases classified into the group of EC 1.1.1. For example, enzymes classified into the group of EC 1.1.1.35 (3-hydroxyacyl-CoA dehydrogenase), EC 1.1.1.36 (acetoacetyl-CoA dehydrogenase), EC 1.1.1.157 (3-hydroxybutanoyl-CoA dehydrogenase), EC 1.1.1.211 (long chain 3-hydroxyacyl-CoA dehydrogenase), or EC 1.1.1.259 (3-hydroxypimeloyl-CoA dehydrogenase) can be exemplified as enzymes that can have an activity for the present conversion. The enzyme used in the present invention is not limited as long as it has an activity for the present conversion, and for example, Escherichia coli-derived PaaH consisting of an amino acid sequence set forth in SEQ ID NO: 13 is used (Fig. 2).

In the conversion of Step C in Fig. 1 (3-hydroxyadipyl-CoA dehydratase), 3-hydroxyadipyl-CoA is converted into 2,3-dehydroadipyl-CoA. Examples of the enzyme that can catalyze the present conversion include hydrolyases classified into the group of EC 4.2.1. For example, enzymes classified into the group of EC 4.2.1.17 (enoyl-CoA hydratase), EC 4.2.1.55 (3-hydroxybutanoyl-CoA dehydratase), or EC 4.2.1.74 (long chain enoyl-CoA hydratase) can be exemplified as enzymes that can have an activity for the present conversion. The enzyme used in the present invention is not limited as long as it has an activity for the present conversion, and for example, Escherichia coli-derived PaaF consisting of an amino acid sequence set forth in SEQ ID NO: 14 is used (Fig. 2).

In the conversion of Step D in Fig. 1 (2,3-dehydroadipyl-CoA reductase), 2,3-dehydroadipyl-CoA is converted into adipyl-CoA. Examples of the enzyme that can catalyze the present conversion include oxidoreductases classified into the group of EC 1.3.1. For example, enzymes classified into the group of EC 1.3.1.8 (acyl-CoA dehydrogenase (NADP⁺)), EC 1.3.1.9 (enoyl-ACP reductase (NADH)), EC 1.3.1.38 (trans-2-enoyl-CoA reductase (NADP⁺)), EC 1.3.1.44 (trans-2-enoyl-CoA reductase (NAD⁺)), EC 1.3.1.86 (crotonyl-CoA reductase), EC 1.3.1.93 (long chain acyl-CoA reductase), or EC 1.3.1.104 (enoyl-ACP reductase (NADPH)) can be exemplified as enzymes that can have an activity for the present conversion.

Examples of the 2,3-dehydroadipyl-CoA reductase used in the present invention include enzymes derived from any species selected from Candida auris, Kluyveromyces marxianus, Pichia kudriavzevii, Thermothelomyces thermophilus, Thermothielavioides terrestris, Chaetomium thermophilum, Podospora anserina, Purpureocillium lilacinum, and Pyrenophora teres. Preferably, a Thermothelomyces thermophilus-derived enzyme consisting of an amino acid sequence set forth in SEQ ID NO: 15, a Chaetomium thermophilum-derived enzyme consisting of an amino acid sequence set forth in SEQ ID NO: 16, and a Candida tropicalis-derived enzyme consisting of an amino acid sequence set forth in SEQ ID NO: 17 are used (Fig. 2).

In the conversion of Step E in Fig. 1, adipyl-CoA is converted into adipic acid. Examples of the enzyme that can catalyze the present conversion include thioester hydratases classified into the group of EC 3.1.2. For example, enzymes classified into the group such as EC 3.1.2.1 (acetyl-CoA hydratase) or EC 3.1.2.20 (acyl-CoA hydratase) can be exemplified as enzymes that can have an activity for the present conversion.

In addition, examples of another enzyme that can catalyze the conversion of Step E in Fig. 1 include CoA-transferases classified into the group of EC 2.8.3. For example, enzymes classified into the group of EC 2.8.3.5 (3-oxoacid CoA-transferase), EC 2.8.3.6 (3-oxoadipate CoA-transferase), or EC 2.8.3.18 (succinyl-CoA:acetate CoA-transferase) can be exemplified as enzymes that can have an activity for the present conversion.

Furthermore, examples of conversion of another enzyme that can catalyze the reaction of Step E in Fig. 1 include a pathway that transfers the adipyl group of adipyl-CoA to a phosphate to form an adipyl phosphate by phosphate acyltransferases classified into the group of EC 2.3.1 and then undergoes dephosphorylation by phosphotransferases classified into group EC 2.7.2. For example, enzymes classified into the group of EC 2.3.1.8 (phosphate acetyltransferase) or EC 2.3.1.19 (butyryl phosphate transferase) as an acyltransferase and enzymes classified into the group of EC 2.7.2.1 (acetate kinase) or EC 2.7.2.7 (butanoate kinase) as phosphotransferases can be exemplified as enzymes that can have an activity for the present conversion.

In the conversion of Step F in Fig. 1, adipyl-CoA is converted into an adipate semialdehyde. Examples of the enzyme that can catalyze the present conversion include enzymes classified into the group of EC 1.2.1. For example, enzymes classified into the group of EC 1.2.1.10 (acetaldehyde dehydrogenase (acetylation)), EC 1.2.1.17 (glyoxylate dehydrogenase (acylation)), EC 1.2.1.42 (hexadecanal dehydrogenase (acylation)), EC 1.2.1.44 (cinnamoyl-CoA reductase (acylation)), EC 1.2.1.75 (malonyl-CoA reductase (malonic semialdehyde formation)), or EC 1.2.1.76 (succinic semialdehyde dehydrogenase (acylation)) can be exemplified as enzymes that can have an activity for the present conversion because these enzymes catalyze a conversion reaction in which CoA is removed and an aldehyde is produced similarly to the present conversion. The enzyme used in the present invention is not limited as long as it has an activity for the present conversion, and for example, Clostridium kluyveri-derived sucD consisting of an amino acid sequence set forth in SEQ ID NO: 18 is used (Fig. 2).

In the conversions of Steps G, I, K, and N in Fig. 1, a carboxyl group is converted into an aldehyde. Examples of the enzyme that can catalyze the present conversion include a carboxylic acid reductase (CAR). For example, enzymes classified into the group of EC 1.2.1.30 (carboxylic acid reductase (NADP⁺)), EC 1.2.1.31 (L-aminoadipate semialdehyde dehydrogenase), EC 1.2.1.95 (L-2-aminoadipate reductase), or EC 1.2.99.6 (carboxylic acid reductase) can be exemplified as enzymes that can have an activity for the present conversion because these enzymes catalyze a conversion reaction in which an aldehyde is generated from a carboxylic acid similarly to the present conversion. Typical examples of the biological species from which the enzyme is derived include, but are not limited to, Nocardia iowensis, Nocardia asteroides, Nocardia brasiliensis, Nocardia farcinica, Segniliparus rugosus, Segniliparus rotundus, Tsukamurella paurometabola, Mycobacterium marinum, Mycobacterium neoaurum, Mycobacterium abscessus, Mycobacterium avium, Mycobacterium chelonae, Mycobacterium immunogenum, Mycobacterium smegmatis, Serpula lacrymans, Heterobasidion annosum, Coprinopsis cinerea, Aspergillus flavus, Aspergillus terreus, Neurospora crassa, and Saccharomyces cerevisiae. The enzyme that can be used in the present invention is not limited as long as it has an activity for the present conversion, and for example, at least one of enzyme MaCar derived from Mycobacterium abscessus consisting of an amino acid sequence set forth in SEQ ID NO: 19 and MaCar(m), which is a variant of MaCar, consisting of an amino acid sequence set forth in SEQ ID NO: 20 is used, and MaCar(m) consisting of an amino acid sequence set forth in SEQ ID NO: 20 is more preferably used (Fig. 2).

In addition, the carboxylic acid reductase can be converted into an active holoenzyme by being phosphopantetheinylated (Venkitasubramanian et al., Journal of Biological Chemistry, Vol. 282, No. 1, 478-485 (2007)). The phosphopantetheinylation is catalyzed by a phosphopantetheinyl transferase (PT). Examples of the enzyme that can catalyze the present reaction include enzymes classified into EC 2.7.8.7. Therefore, the microorganism of the present invention may be further modified to increase an activity of the phosphopantetheinyl transferase. Examples of a method of increasing the activity of the phosphopantetheinyl transferase include, but are not limited to, a method of introducing an exogenous phosphopantetheinyl transferase and a method of enhancing expression of an endogenous phosphopantetheinyl transferase. The enzyme used in the present invention is not particularly limited as long as it has a pheosphopantetheinyl group transfer activity, and examples thereof include EntD of Escherichia coli, Sfp of Bacillus subtilis, Npt of Nocardia iowensis (Venkitasubramanian et al., Journal of Biological Chemistry, Vol. 282, No.1, 478-485 (2007)), and Lys5 of Saccharomyces cerevisiae (Ehmann et al., Biochemistry 38.19 (1999): 6171-6177). The enzyme used in the present invention is not limited as long as it has an activity for the present conversion, and for example, Nocardia iowensis-derived Npt consisting of an amino acid sequence set forth in SEQ ID NO: 21 is used (Fig. 2).

The conversions of Steps J, M, P, and R in Fig. 1 are transamination reactions. Examples of the enzyme that can catalyze the conversion include a transaminase (aminotransferase) classified in the group of EC 2.6.1. For example, enzymes classified into the group of EC 2.6.1.19 (4-aminobutanoate-2-oxoglutarate transaminase), EC 2.6.1.29 (diamine transaminase), or EC 2.6.1.48 (5-aminovalerate transaminase) can be exemplified as enzymes that can have an activity for the present conversion. The enzyme used in the present invention is not particularly limited as long as it has the conversion activity of each step, for example, YgjG that is putrescine aminotransferase of Escherichia coli reported to transaminate cadaverine and spermidine (Samsonova., et al., BMC microbiology 3.1 (2003): 2), SpuC that is putrescine aminotransferase of the genus Pseudomonas (Lu et al., Journal of bacteriology 184.14 (2002): 3765-3773, Galman et al., Green Chemistry 19.2 (2017): 361-366), GABA aminotransferase GabT of Escherichia coli, and PuuE may also be used. Furthermore, it is reported that an ω-transaminase derived from a biological species such as Ruegeria pomeroyi, Chromobacterium violaceum, Arthrobacter citreus, Sphaerobacter thermophilus, Aspergillus fischeri, Vibrio fluvialis, Agrobacterium tumefaciens, or Mesorhizobium loti also has a transamination activity to a diamine compound such as 1,8-diaminooctane or 1,10-diaminodecane, and these enzymes may be used in the present invention (Sung et al., Green Chemistry 20.20 (2018): 4591-4595, Sattler et al., Angewandte Chemie 124.36 (2012): 9290-9293). The enzyme used in the present invention is not limited as long as it has an activity for the present conversion, and for example, Escherichia coli-derived YgjG consisting of an amino acid sequence set forth in SEQ ID NO: 22 may be used (Fig. 2). Examples of a typical amino group donor include, but are not limited to, L-glutamic acid, L-alanine, and glycine.

The genes encoding the aforementioned enzymes that can be used in the present invention may be derived from a microorganism other than the exemplified microorganism or artificially synthesized, and may be any gene that can express a substantial enzymatic activity in the host microorganism cell.

In addition, the enzyme gene that can be used for the present invention may have all naturally occurring mutations and artificially introduced mutations and modifications as long as it can express a substantial enzymatic activity in the host microorganism cell. For example, it is known that extra codons are present in various codons encoding a specific amino acid. Therefore, in the present invention, alternative codons that are to be finally translated into the same amino acid may also be used. That is, since a genetic code degenerates, a plurality of codons can be used to encode a specific amino acid, such that the amino acid sequence can be encoded by an arbitrary one set of similar DNA oligonucleotides. Only the member of the set is identical to the gene sequence of the natural enzyme; however, even mismatched DNA oligonucleotides can hybridize to natural sequences under appropriate stringent conditions (for example, hybridize at 3xSSC and 68°C and wash at 2xSSC and 0.1%SDS and 68°C), DNA encoding a natural sequence can by identified and isolated, and such a gene can also be used in the present invention. In particular, since most organisms are known to preferentially use a subset of a specific codon (optimal codon) (Gene, Vol. 105, pp. 61-72, 1991, and the like), performing of "codon optimization" depending on a host microorganism can also be useful in the present invention.

In a case where the diamine is 1,5-pentamethylenediamine, in a preferred aspect, the recombinant microorganism contains a base sequence having a homology of 85% or more, 90% or more, 92% or more, 95% or more, 98% or more, or 99% or more and preferably a homology of 92% or more, 95% or more, 98% or more, or 99% or more with a base sequence set forth in SEQ ID NO: 2 or 4, or the recombinant microorganism contains a base sequence having a homology of 85% or more, 90% or more, 92% or more, 95% or more, 98% or more, or 99% or more and preferably a homology of 92% or more, 95% or more, 98% or more, or 99% or more with a base sequence encoding an amino acid sequence set forth in SEQ ID NO: 1 or 3.

For example, when the diamine synthase gene is introduced into a host microorganism cell as an "expression cassette", a stable and high level of enzymatic activity can be obtained. Preferably, when a gene sequence of the "expression cassette" is inserted into the genome sequence of the host microorganism, a more stable and high level of enzymatic activity can be obtained.

Here, in the present specification, the "expression cassette" refers to a nucleotide having a nucleic acid sequence that is functionally linked to a nucleic acid to be expressed or a gene to be expressed and regulates transcription and translation. Typically, the expression cassette of the present invention contains a promoter sequence on the 5' upstream from the coding sequence, a terminator sequence on the 3' upstream, and an optionally additional normal regulatory element in a functionally linked state, and in such a case, a nucleic acid to be expressed or a gene to be expressed is introduced into a host microorganism.

In addition, the promoter is defined as a DNA sequence that allows RNA polymerase to bind to DNA to initiate RNA synthesis, regardless of whether the promoter is a constitutive expression promoter or an inductive expression promoter. A strong promoter is a promoter that initiates mRNA synthesis at a high frequency, and is also preferably used in the present invention. For example, in Bacillus pseudofirmus, a promoter region for a synthetase of SLayer protein, a sigma factor (for example, rpoD or the like), a glycolytic enzyme (for example, glyceraldehyde-3-phosphate dehydrogenase), a lactate dehydrogenase, and glutamic acid decarboxylase A, and the like can be used.

The expression cassette described above is incorporated into a vector consisting of, for example, a plasmid, a phage, a transposon, an IS element, a fosmid, a cosmid, or a linear or cyclic DNA and is introduced into a host microorganism. In the present invention, a plasmid and a phage are preferable. These vectors may be self-replicated in a host microorganism or may be replicated by being inserted into a chromosome. Examples of a preferred plasmid include pUB 110, pC 194, and pBD 214 for bacillus such as the genus Bacillus.

The expression cassette described above is preferably inserted into a chromosome as compared to a plasmid and a phage. In order to retain the plasmid in the host microorganism, a certain selective pressure is required, and generally, addition of an antibiotic corresponding to an antibiotic resistance marker of the plasmid to the medium is required. In addition, even at a selection pressure, in a case where a gene expressed on the plasmid is not required for the host microorganism or becomes a burden on growth, there is a possibility that a mutation is introduced or deleted on the gene by the action of an enzyme endogenously retained by the host microorganism, and it is often difficult to stably produce a substance.

Examples of a usable plasmid and the like include those described in "Cloning Vectors", Elsevier, 1985, in addition to those described above. The expression cassette can be introduced into the vector by a conventional method including fragment amplification by PCR, cutting with an appropriate restriction enzyme, cloning, and various ligations.

As a method that can be applied when a vector containing the expression cassette of the present invention is constructed as described above and then the vector is introduced into a host microorganism, for example, conventional cloning methods and transfection methods such as conjugative transfer, coprecipitation, protoplast fusion, electroporation, and retroviral transfection are used. Examples thereof are described in "Current Protocols in Molecular Biology", F. Ausubel et al., Publ. Wiley Interscience, New York, 1997, or Sambrook et al., and "Molecular Cloning: Laboratory Manual", 2nd ed., Cold Spring Harbor Laboratory Press, Cold Spring Harbor, NY, 1989.

Furthermore, a temperature-sensitive replication origin sequence, a negative selection gene, and homologous sequences on both sides of a region to be edited of the genome sequence of the host microorganism are included in the sequence of the plasmid vector, such that an exogenous gene can be introduced into the genome sequence of the host microorganism, or unnecessary genes can be removed. Preferably, the temperature-sensitive replication origin sequence is the replication origin used in the pE194ts vector, and the negative selection gene is the sacB gene or the mutant pheS gene.

In the process of culturing the host microorganism into which the vector described above is introduced, it is possible to select an edited cell whose genome sequence is modified by a change in temperature and a change in medium composition. As conditions for selecting the edited cell, it is preferable that the culture temperature is 37 to 43°C or higher, the medium contains 10% or more of sucrose, or the medium contains 1 mM or more of 4-chlorophenylalanine.

In a case where the diamine to be produced is 1,5-pentamethylenediamine, in a preferred aspect, the host microorganism is further modified by a mutation manipulation or a genetic recombination manipulation to improve a lysine producing ability, that is, the obtained recombinant microorganism further contains a modification by a mutation manipulation or a genetic recombination manipulation to improve a lysine producing ability. The mutation manipulation or the genetic recombination manipulation is, for example, a manipulation of canceling a feedback inhibition on at least one of aspartokinase III (EC 2.7.2.4) and 4-hydroxy-tetrahydrodipicolinate synthase (EC 4.3.3.7).

Aspartokinase III is an enzyme that catalyzes the reaction to convert aspartic acid and adenosine triphosphate (ATP) into 4-phospho-aspartic acid and adenosine diphosphate. It is generally recognized that the present enzyme is subjected to feedback inhibition by L-lysine. In a mutant enzyme modified so as not to be subjected to feedback inhibition, the protein structure changes, L-lysine does not bind, and the mutant enzyme has an enzymatic activity even in the presence of lysine. Therefore, it is known that a microorganism expressing the present enzyme produces lysine at a high level. Hereinafter, aspartokinase III (lysC) derived from Eschericia coli will be described as an example of an effective mutation, but the gene used in the present invention is not limited thereto. Examples of the mutant lysC that is not subjected to feedback inhibition by L-lysine include, but are not limited to, those in which a threonine residue at position 352 in the amino acid sequence is substituted with an isoleucine residue, and those in which a threonine residue at position 253 is substituted with an arginine residue.

4-Hydroxy-tetrahydrodipicolinate synthase is an enzyme that catalyzes the reaction of converting pyruvic acid and aspartate semialdehyde into (2S,4S)-4 hydroxy-2,3,4,5-tetrahydro-(2S)-dipicolinate and water. It is generally recognized that the present enzyme is subjected to feedback inhibition by L-lysine. In a mutant enzyme modified so as not to be subjected to feedback inhibition, the protein structure changes, L-lysine does not bind, and the mutant enzyme has an enzymatic activity even in the presence of lysine. Therefore, a microorganism expressing the enzyme is known to produce lysine at a high level. Hereinafter, 4-hydroxy-tetrahydrodipicolinate synthase (dapA) derived from Eschericia coli will be described as an example of an effective mutation, but the gene used in the present invention is not limited thereto. Examples of the mutant dapA that is not subjected to feedback inhibition by L-lysine include, but are not limited to, those in which an alanine residue at position 81 in the amino acid sequence is substituted with a valine residue, those in which a glutamic acid residue at position 84 is substituted with a threonine residue, and those in which a histidine residue at position 118 is substituted with an arginine residue or a tyrosine residue.

The transformant or genome-edited cell obtained as described above is cultured and maintained under conditions preferred for the growth and/or maintenance of the transformant in order to produce a diamine. For example, in a case where 1,5-pentanediamine is produced, a transformant transformed with a vector (each expression cassette may be located on a separate or the same vector) containing an expression cassette of an exogenous L-lysine decarboxylase gene, or a genome-edited cell in which an expression cassette of an exogenous L-lysine decarboxylase gene is integrated into a genome sequence of a host microorganism is cultured and maintained under conditions suitable for growth and/or maintenance of the transformant to produce 1,5-pentanediamine. Suitable medium composition, culture conditions, and culture time for transformants derived from various host microorganism cells can be easily set by those skilled in the art.

The medium may be a natural, semi-synthetic, or synthetic medium containing one or more carbon sources, nitrogen sources, inorganic salts, vitamins, and optionally a trace component such as trace element or vitamin. However, the medium to be used is required to appropriately satisfy nutritional requirements of the transformants to be cultured.

Examples of the carbon source include D-glucose, sucrose, lactose, fructose, maltose, oligosaccharides, polysaccharides, starch, cellulose, rice bran, waste molasses, fats and oils (for example, soybean oil, sunflower oil, peanut oil, palm oil, and the like), fatty acids (for example, palmitic acid, linoleic acid, linolenic acid, and the like), alcohols (for example, glycerol, ethanol, and the like), and organic acids (for example, acetic acid, lactic acid, succinic acid, and the like). Furthermore, the carbon source may be biomass containing D-glucose. Examples of suitable biomass include a corn decomposition liquid and a cellulose decomposition liquid. Furthermore, examples of the other carbon sources include sugar, carbon dioxide, synthetic gas, methanol, and amino acid. These carbon sources can be used alone or as a mixture.

The diamine as a product using a raw material derived from biomass can be clearly distinguished from a synthetic raw material derived from, for example, petroleum, natural gas, or coal, by measurement of a biomass carbon content based on Carbon-14 (radiocarbon) analysis defined in ISO 16620-2 or ASTM D6866.

Examples of the nitrogen source include nitrogen-containing organic compounds (for example, peptone, a yeast extract, a meat extract, a malt extract, a corn steep liquor, soy flour, urea, and the like) and inorganic compounds (for example, ammonium sulfate, ammonium chloride, ammonium phosphate, ammonium carbonate, sodium nitrate, ammonium nitrate, and the like). These nitrogen sources can be used alone or as a mixture.

In addition, the medium may contain a corresponding antibiotic in a case where a transformant expresses a useful additional trait, for example, in a case where a transformant contains a marker resistant to an antibiotic. Therefore, a risk of contamination by various bacteria during fermentation is reduced. Examples of the antibiotic include, but are not limited to, ampicillin, kanamycin, chloramphenicol, tetracycline, erythromycin, streptomycin, and spectinomycin.

In a case where the host microorganism cannot assimilate the carbon sources such as cellulose and polysaccharides, the host microorganism can be adapted to produce a diamine using these carbon sources by subjecting the host microorganism to a known genetic engineering technique such as introduction of an exogenous gene. Examples of the exogenous gene include a cellulase gene and an amylase gene.

The culture may be batch or continuous. In addition, in any case, an additional carbon source or the like may be supplied at an appropriate time of culture. Furthermore, the culture should be continued while maintaining a suitable temperature, oxygen concentration, pH, and the like. A suitable culture temperature of a general transformant derived from a microbial host cell is usually 15°C to 50°C, preferably 25°C to 37°C. In a case where the host microorganism is aerobic, shaking (flask culture or the like) and agitation/aeration (jar fermenter culture or the like) are required to ensure a suitable oxygen concentration during fermentation. These culture conditions can be easily set by those skilled in the art.

In addition, another embodiment of the present invention relates to a method for manufacturing a diamine using the recombinant microorganism described above. The method for manufacturing a diamine includes, for example, the following steps.

### (a) Culture step

The method for manufacturing a diamine includes a culture step of culturing the recombinant microorganism according to the embodiment described above. A culture solution containing bacterial cells is obtained by the culture in this step. In the culture step, the recombinant microorganism may be cultured in a culture solution containing an inorganic salt. The inorganic salt is a hydrochloride, a sulfate, a phosphate, a carbonate, a hydrofluoric acid salt, or the like of a metal element, and examples thereof include sodium chloride, lithium chloride, sodium sulfate, potassium sulfate, magnesium sulfate, and sodium carbonate, and among them, sodium sulfate and sodium carbonate are preferable. Without intending to be limited by any theory, it is considered that when an inorganic salt is added to an aqueous solution, water molecules are fixed as hydrated water by its strong hydration force, such that the amount of water molecules required for hydration of diamines is reduced, and phase separation occurs. There is a Hofmeister series as an index indicating the strength of salting out of an inorganic salt, and a salt including a combination of an anion and a cation (particularly, a metal ion) that strongly causes salting out, which is shown in the Hofmeister series, is preferable. Diamines are produced by culturing in a culture solution containing an inorganic salt, but in the presence of a high concentration of an inorganic salt, phase separation of the diamine aqueous solution occurs from the culture solution. Therefore, it is considered that the separation of the diamine from the culture solution is facilitated by containing the inorganic salt in the culture solution.

In this case, the recombinant composition is cultured in, for example, a culture solution containing 5 mass% or more of an inorganic salt, and preferably cultured in a culture solution containing 10 mass% or more of an inorganic salt. In another aspect, the recombinant composition is cultured in, for example, a culture solution containing 5 to 20 mass%, and preferably 10 to 20 mass% of an inorganic salt.

The phase containing the inorganic salt can be separated from the culture solution by a separation step described below and then reused as a culture solution. Wastewater containing a high concentration of a salt requires cost for treatment, but the cost can be suppressed by reuse. In addition, it is also possible to reduce the load in the purification process, in particular, the load of a dehydration concentration by phase separation. For example, a prior art in which a strong base is added for separation of a diamine has also been proposed. However, in a case where a strong base is added, phase separation caused by addition of an inorganic salt does not occur, and a dehydration concentration ratio from a medium increases in diamine purification, and a solvent extraction step is additionally required. As described above, when the inorganic salt is added to the culture solution, the load of dehydration concentration can be reduced in the purification process, and an additional step for solvent extraction is also not required.

### (b) Reaction step

The present step is a step of bringing a precursor of a diamine into contact with the recombinant microorganism to produce a target diamine from the diamine precursor. The contact with the diamine precursor may be performed, for example, in the culture step or after the step.

For example, in a case where the diamine is 1,5-pentamethylenediamine, the recombinant microorganism is brought into contact with lysine, such that lysine is decarboxylated by lysine decarboxylase produced by the recombinant microorganism to produce 1,5-pentamethylenediamine.

In an aspect, in the present step, the culture solution and/or the bacterial cells obtained in the culture step are brought into contact with an aqueous solution containing 5 mass% or more of an inorganic salt and lysine to obtain a reaction solution containing 1,5-pentanediamine. For example, in the present step, the culture solution containing the bacterial cells obtained in the culture step and/or the bacterial cells from which a supernatant is removed by centrifugation or the like from the culture solution obtained in the culture step are brought into contact with an aqueous solution containing an inorganic salt and lysine to obtain a reaction solution.

In another aspect, the culture step and the reaction step may be performed in the same step. For example, in a case where the diamine is 1,5-pentamethylenediamine, an aqueous solution containing an inorganic salt and lysine may be added to a culture solution for culturing a recombinant microorganism. In addition, for example, bacteria that produce lysine by fermentation and the recombinant microorganism according to the present invention may be co-cultured. By co-culturing these microorganisms, lysine produced by the bacteria can be efficiently converted into 1,5-pentamethylenediamine by lysine decarboxylase produced by the recombinant composition according to the present invention.

### (Addition of inorganic salt)

The inorganic salt may be present in the culture solution in advance. That is, as described in connection with the culture step, the recombinant microorganism according to the present invention may be cultured in a culture solution containing an inorganic salt. In this case, the recombinant composition is cultured in, for example, a culture solution containing 5 mass% or more of an inorganic salt, preferably a culture solution containing 10 mass% or more of an inorganic salt. In another aspect, the recombinant composition is cultured in, for example, a culture solution containing 5 to 20 mass% of an inorganic salt, preferably a culture solution containing 10 to 20 mass% of an inorganic salt.

In a case where an inorganic salt is added to the culture solution, the inorganic salt is added so that a concentration of the inorganic salt in the culture solution is 100 to 200 g/L, preferably 150 to 200 g/L, more preferably 160 to 200 g/L, and still more preferably 200 g/L.

Alternatively, in a case where the diamine is 1,5-pentamethylenediamine, the inorganic salt may be brought into contact with the culture solution and/or bacterial cells obtained in the culture step as an aqueous solution together with lysine in the reaction step. In an aspect, the inorganic salt may be added in both the culture step and the reaction step. In another aspect, the inorganic salt may be added in one or more steps in the method for manufacturing a diamine as described below in addition to the culture step and/or the reaction step.

The inorganic salt is sodium carbonate or sodium sulfate, more preferably sodium sulfate.

By adding the inorganic salt, the phase containing a diamine and the aqueous phase containing an inorganic salt can be phase-separated in a separation step described below, and the diamine is easily separated. In addition, in a case where the recombinant microorganism according to the present invention is a microorganism having halophilicity, the growth of the microorganism is not inhibited even in a case where a high concentration of the inorganic salt is added as described above, and thus there is an advantage that the separation of the phase containing a diamine can be promoted without hindering the production of the diamine, and the diamine can be easily isolated.

### (c) Removal step

The manufacturing method may further include a removal step of removing the recombinant microorganism from the culture solution or the reaction solution. The step is performed by, for example, centrifugation and/or filtration after a diamine is produced by the culture step or the reaction step. By this step, a solid content such as bacterial cells contained in the culture solution or the reaction solution can be removed. In addition, a polymer compound having an arbitrary molecular weight or more containing polysaccharides, proteins, and the like can be removed by using an ultrafiltration membrane at the time of filtration treatment.

### (d) Concentration step

In this step, the culture solution or the reaction solution is concentrated. The concentration step is performed, for example, by removing the recombinant microorganism by the removal step and then concentrating the culture supernatant using, for example, an evaporator. By concentrating the culture solution containing a diamine, it can be expected that the concentrations of the diamine and the inorganic salt increase and the separation efficiency is further improved.

In a case where the diamine is one or more forms selected from the group consisting of a carbonate salt, a bicarbonate salt, a bisbicarbonate salt, a carbamate salt, and a biscarbamate salt produced by the culture step and/or the reaction step, in the concentration step, the diamine in the forms of the salts is converted into a free base and carbon dioxide, and the carbon dioxide is separated. For example, in a case where the diamine is 1,5-pentamethylenediamine, in the concentration step, 1,5-pentamethylenediamine that is one or more forms selected from the group consisting of a carbonate salt, a bicarbonate salt, a bisbicarbonate salt, a carbamate salt, and a biscarbamate salt produced by the culture step is converted into a free base and carbon dioxide, and the carbon dioxide is separated.

### (e) pH adjustment step

In the pH adjustment step, a pH of the culture solution or the reaction solution is adjusted to 12 or higher. For example, in the present step, the culture solution or the reaction solution is concentrated by the concentration step, and then a pH of the concentrated culture solution or reaction solution is adjusted to 12 or higher. Alternatively, it is confirmed that the pH is 12 or higher by pH increase due to the separation of carbon dioxide in the concentration step.

### (f) Separation step

In the present step, the phase containing a diamine is separated from the culture solution or the reaction solution. The separation step does not include adding an alkaline compound. The alkaline compound referred to herein refers to a compound whose aqueous solution exhibits basicity and has an action of increasing a pH value by adding the compound, and is particularly an inorganic alkaline compound. A hydroxide of a metal element and an inorganic substance capable of accepting hydrogen ions correspond to the alkaline compound, and examples thereof include potassium hydroxide, sodium hydroxide, calcium hydroxide, lithium hydroxide, ammonia, magnesium hydroxide, aluminum hydroxide, manganese hydroxide, iron hydroxide, cobalt hydroxide, copper hydroxide, zinc hydroxide, and barium hydroxide. Since the alkaline compound is not added, a reduction in cost is achieved by not using the alkaline compound except in the medium component. In addition, since a pH of the phase containing an inorganic salt after the diamines resulting from a high pH are separated is lowered, it is also possible to recycle the phase as a medium. In a case where an inorganic salt is contained in the culture solution or the reaction solution, in the present separation step, a phase containing a diamine and an aqueous phase containing an inorganic salt are phase-separated.

The phase separation may be performed by adding an organic solvent to the culture solution or the reaction solution, or by bringing the culture solution or the reaction solution into contact with the organic solvent. The organic solvent is one or more selected from the group consisting of n-hexane, n-butanol, and 2-ethyl-1-hexanol. For example, in a case where the diamine is 1,5-pentamethylenediamine, n-butanol is preferably used. In a case where the diamine is HMDA, 2-ethyl-1-hexanol is preferably used.

As described above, phase separation of diamines is promoted by an inorganic salt having a high salt concentration (for example, sodium carbonate and sodium sulfate), but phase separation may be further promoted by adding an organic solvent. By adding the organic solvent, the extraction of the diamine from the aqueous phase to the organic phase can be promoted to increase the transfer rate to the organic phase, and the yield can be further improved.

### (g) Aqueous phase recovery step and reusing step

In the present step, a phase containing an inorganic salt (including, for example, an inorganic salt and water) is recovered and/or the recovered phase is reused as a culture solution. Since the recombinant microorganism according to the present invention has halophilicity, a diamine can be produced without inhibiting growth even in a culture solution containing an inorganic salt. In addition, since phase separation is promoted by containing an inorganic salt, the diamine is easily separated. Furthermore, there is a possibility that the treatment of water containing an inorganic salt at a high concentration will be costly, but the wastewater treatment cost can be reduced by reusing the water as a medium for a microorganism as described above.

### (h) Purification step

In the present step, the diamine obtained from the culture is purified. A method for purifying a diamine, for example, 1,5-pentanediamine, from a culture is known to those skilled in the art. In a case of a transformant or a genome-edited cell or of a prokaryotic microorganism host cell, 1,5-pentanediamine is present in the culture supernatant or in the bacterial cells, but may be extracted from the cultured bacterial cells if necessary. In the case of extracting from the cultured bacterial cells, for example, the culture is centrifuged to separate the supernatant and the bacterial cells, and the bacterial cells can be disrupted by a surfactant, an organic solvent, an enzyme, or the like while using a homogenizer. As a method for purifying a diamine from the culture supernatant, and if necessary, from the bacterial cell extract, deproteinization using protein precipitation by pH adjustment or the like, removal by adsorption of impurities using activated carbon, and removal by adsorption of ionic substances using an ion exchange resin or the like are performed, and then purification is performed by extraction, distillation, or the like using a known solvent. It goes without saying that some steps may be omitted depending on the purity intended by the product, or an additional purification step such as chromatography may be performed.

Still another embodiment of the present invention also relates to a method for purifying a diamine from a culture obtained using the recombinant composition described above. The purification method may include each of the above steps, which are described for the method for manufacturing a diamine, alone or in combination.

In general, the growth of Escherichia coli and coryneform bacteria used in the conventional method is significantly inhibited in an environment with a pH of 9 or higher. Therefore, for example, when the pH in the medium increases by converting lysine into 1,5-pentamethylenediamine, it is required to control the pH to a range in which the growth of microorganisms is not inhibited by appropriately adding an acid solution. However, since the recombinant microorganism according to the present invention has alkalinity capable of growing even in an alkaline environment, it is not required to adjust the pH by adding an acid solution as in the prior art by using the microorganism of the present invention for diamine production, and complication of the diamine production process can be avoided. In addition, in a case where the recombinant microorganism according to the present invention is halophilic, the recombinant microorganism can also grow in a culture solution containing a salt. Therefore, since the waste liquid containing a salt added in the separation step can be reused for culturing the microorganism, the wastewater treatment cost can be reduced. In addition, compared to the prior art in which a strong base is added for diamine separation, when the inorganic salt is added to the culture solution, the load of dehydration concentration can be reduced in the purification process, and an additional step for solvent extraction is also not required.

### <2> Invention B: Halophilic and/or Alkalophilic Recombinant Microorganism Having Suppressed N-Acetyldiamine Production

The genetically modified microorganism of the present invention is a halophilic and/or alkalophilic host microorganism having a diamine producing ability and containing one or more genetic modifications to suppress an N-acetylation enzyme.

In natural world, there are microorganisms having alkalinity capable of growing in an alkaline environment with a pH of 9 or higher, halophilicity capable of growing in an environment with a NaCl concentration of 0.2 M or higher, or both of these properties. In relation to the present invention, an alkalophilic microorganism that can be used as a host microorganism is a kind of an extreme environmental microorganism exhibiting various distributions, and is a generic term for microorganisms that can grow even in an environment with a pH of 9 or higher. These microorganisms are classified into absolutely alkalophilic microorganisms capable of growing only in an environment with a pH of 9 or higher and facultative alkalophilic microorganisms capable of growing even at a pH of lower than 9 although having an optimum growth at a pH of 9 or higher. Some of them can also grow in a strongly alkaline environment with a pH of 12 or higher. All of these microorganisms are alkalophilic microorganisms, and can be host microorganisms in the present invention.

In relation to the present invention, the halophilic microorganism that can be used as a host microorganism is a generic term for microorganisms that can respond to high-concentration salt stress. In the classification of bacteria by an optimal growth salt concentration, these bacteria are classified into non-halophilic bacteria in which an optimal growth salt concentration of sodium chloride is 0 to 0.2 M, low-halophilic bacteria in which an optimal growth salt concentration of sodium chloride is 0.2 to 0.5 M, medium-halophilic bacteria in which an optimal growth salt concentration of sodium chloride is 0.5 to 2.5 M, and highly halophilic bacteria in which an optimal growth salt concentration of sodium chloride is 2.5 to 5.2 M. All of these microorganisms are halophilic microorganisms, and can be host microorganisms in the present invention.

Examples of the microorganism having halophilicity, alkalophilicity, or both properties that can be used as the host microorganism of the present invention include various microorganisms, and non-limiting examples thereof include bacteria of the genus Bacillus, the genus Halomonas, the genus Halobacteroides, the genus Salinibacter, the genus Alkaliphilus, the genus Clostridium, and the genus Anaerobranca. The host microorganism of the present invention is preferably a bacterium of the genus Bacillus. Among the microorganisms of the genus Bacillus, Bacillus pseudofirmus, Bacillus halodurans, and Bacillus marmarensis are preferable, and Bacillus psuedofirmus is more preferable.

The growth of Escherichia coli and coryneform bacteria generally used for compound biosynthesis is significantly inhibited in an environment with a pH of 9 or higher. Therefore, for example, when the pH in the medium increases by converting lysine into 1,5-pentamethylenediamine, it is required to control the pH to a range in which the growth of microorganisms is not inhibited by appropriately adding an acid solution. However, since the recombinant microorganism according to the present invention has alkalinity capable of growing even in an alkaline environment, it is not required to adjust the pH by adding an acid solution as in the related art by using the microorganism for diamine production, and complication of the diamine production process can be avoided. In addition, in a case where the recombinant microorganism according to the present invention is halophilic, the recombinant microorganism can grow also in a culture solution containing a salt. Therefore, since the waste liquid containing a salt added in the separation step of separating the phase containing a diamine from the culture solution or the mixed solution (reaction solution) can be reused for culturing the microorganism according to the present invention, the wastewater treatment cost can be reduced.

One or more genetic modifications that suppress an N-acetylation enzyme are performed by using the halophilic and/or alkalophilic microorganism having a diamine producing ability as a host microorganism, such that in the obtained recombinant microorganism, it is possible to suppress production of N-acetyldiamine as a by-product.

That is, the production of N-acetyldiamine is suppressed in the halophilic and/or alkalophilic microorganism having a diamine producing ability, such that the production of N-acetyldiamine can be suppressed in the enzyme conversion step or the culture step. In a preferred aspect, the diamine compound as a target compound can be efficiently produced by suppressing production of a by-product.

In the present specification, the "N-acetylation enzyme" is an enzyme that N-acetylates a diamine compound to produce an N-acetyldiamine compound. The term "N-acetylation enzyme" is synonymous with the terms "N-acetyltransferase" and "N-acetyltransferase", and these terms are used interchangeably in the present specification. The N-acetylation enzyme is an enzyme that catalyzes a reaction of acetylating the N-terminus of an amino acid, and catalyzes a reaction of N-acetylating a diamine to produce N-acetyldiamine as a side reaction in a diamine production pathway.

In the present invention, as described above, in the halophilic and/or alkalophilic host microorganism, one or more genetic modifications that suppress the N-acetylation enzyme are performed, and thus the recombinant microorganism according to the present invention contains one or more genetic modifications that suppress the N-acetylation enzyme. The genetic modification is:
- a modification that suppresses expression of an endogenous gene encoding the N-acetylation enzyme; or
- a modification that reduces an activity of the N-acetylation enzyme.

The suppression of the N-acetylation enzyme in the microorganism can be confirmed using, for example, an analysis method such as ion chromatography, by at least one of:
- no detection of an N-acetyl derivative in the culture supernatant of the microorganism;
- no decrease in cadaverine as a substrate during enzymatic activity measurement using a cell lysate; and
- no detection of an N-acetyl derivative as a product.

In a preferred aspect, in the recombinant microorganism according to the present invention, an N-acetyldiamine compound producing ability is suppressed or eliminated as compared with the producing ability of a non-mutant strain that does not contain the genetic modification.

One or more genetic modifications that suppress the N-acetylation enzyme are modifications for suppressing the N-acetyldiamine biosynthesis pathway. The genetic modification is performed, for example, by one or more of:
· deletion of a part or the entire of the endogenous gene encoding an N-acetylation enzyme in a host microorganism from a genome sequence of the host microorganism;
· introduction of a mutation that causes a loss of an enzymatic function in the genome sequence of the N-acetylation enzyme in the host microorganism; and
· introduction of a mutation such as substitution, insertion, or deletion into a promoter site and/or an RBS site of the N-acetylation enzyme.

The modification for suppressing the N-acetyldiamine biosynthetic pathway is performed, for example, by one or more of:
· deletion of a part or the entire of the endogenous gene encoding an N-acetylation enzyme in a host microorganism from a genome sequence of the host microorganism;
· introduction of a mutation that causes a loss of an enzymatic function in the genome sequence of the N-acetylation enzyme in the host microorganism; and
· introduction of a mutation such as substitution, insertion, or deletion into a promoter site and/or an RBS site of the N-acetylation enzyme.

In the recombinant microorganism obtained by the above manipulation, production of the N-acetylation enzyme is suppressed. That is, the recombinant microorganism according to the present invention contains modification by one or more genetic manipulations to suppress the production of the N-acetylation enzyme. Due to such modifications, in the recombinant microorganism according to the present invention, preferably, an N-acetyldiamine compound producing ability is suppressed or eliminated as compared with the ability to produce a non-mutant strain that does not contain the genetic modification.

Specifically, the genetic manipulation may be, for example, one or more genetic manipulations selected from the group consisting of the following (A), (B), (C), and (D):
(A) a manipulation of deleting an endogenous gene encoding the N-acetyltransferase in the host microorganism;
(B) a manipulation of decreasing a copy number of the endogenous gene for the N-acetyltransferase in the host microorganism;
(C) a manipulation of introducing a mutation into an expression regulatory region of the endogenous gene for the N-acetyltransferase in the host microorganism; and
(D) a manipulation of replacing the expression regulatory region of the endogenous gene for the N-acetyltransferase in the host microorganism by an exogenous regulatory region which enables low expression.

For the genetic modification, for example, a technique of introducing an arbitrary exogenous gene sequence into a genome sequence in a cell of a host microorganism, and a technique of removing an unnecessary gene sequence from the genome sequence can be used.

Specifically, the following techniques can be exemplified:
(1) a technique of arbitrarily modifying a gene sequence on a chromosome by combining a temperature-sensitive plasmid vector and negative selection; and
(2) a technique of arbitrarily modifying the gene sequence on the chromosome by CRISPR/CAS9.

In the process of culturing the host microorganism into which the vector described above is introduced, it is possible to obtain a genetically modified strain in which the gene sequence of the chromosome is modified through homologous recombination by a change in temperature and a change in medium composition. As conditions for obtaining a genetically modified strain, it is preferable that the culture temperature is 37 to 43°C or higher, and the medium contains 1 mM or more of 4-chlorophenylalanine.

As a method that can be applied when the vector described above is introduced into a host microorganism, for example, conventional cloning methods and transfection methods such as conjugative transfer, coprecipitation, protoplast fusion, electroporation, and retroviral transfection are used. Examples thereof are described in Gene Cloning and DNA Analysis, T.A. Brown, 2016, or Molecular Cloning: A Laboratory Manual (Fourth Edition): Sambrook et al., 2012.

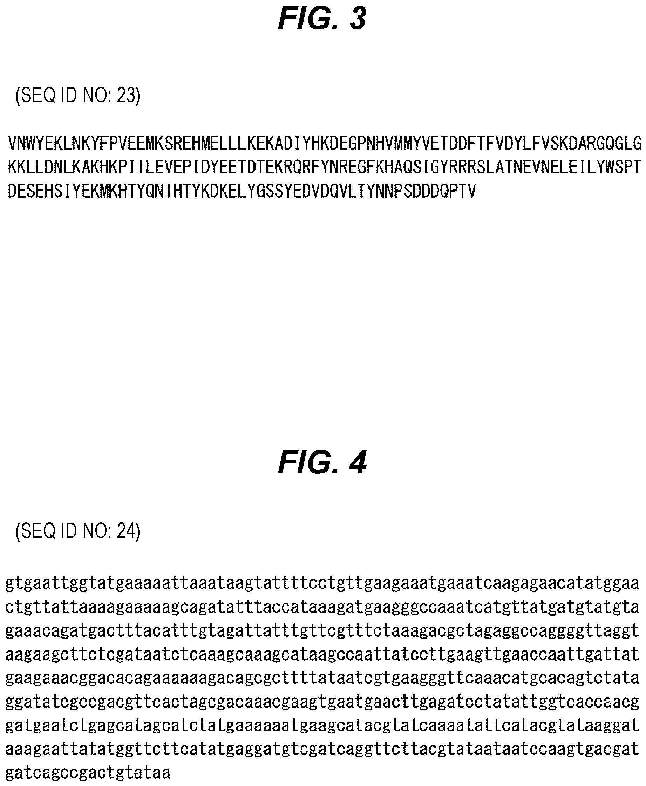

Examples of a representative gene for N-acetyltransferase include yjbC of Bacillus psuedofirmus. An amino acid sequence of the Bacillus psuedofirmus yjbC enzyme is set forth in SEQ ID NO: 23, and a base sequence of Bacillus psuedofirmus yjbC gene (BpOF4_01925 (GenBank: ADC48452)) is set forth in SEQ ID NO: 24 (Figs. 3 and 4).

In an aspect, the N-acetylation enzyme:
(A-1) consists of an amino acid sequence set forth in SEQ ID NO: 23;
(A-2) consists of an amino acid sequence having a sequence identity of 80% or more, 85% or more, 88% or more, 90% or more, 93% or more, 95% or more, 97% or more, 98% or more, or 99% or more with the amino acid sequence set forth in SEQ ID NO: 23, and has an enzymatic activity to N-acetylate a diamine compound to produce an N-acetyldiamine compound; or
(A-3) consists of an amino acid sequence in which 1 to 10, 1 to 7, 1 to 5, or 1 to 3 amino acids are deleted, substituted, inserted, and/or added in the amino acid sequence set forth in SEQ ID NO: 23, and has an enzymatic activity to N-acetylate a diamine compound to produce an N-acetyldiamine compound.

In a preferred aspect, the N-acetylation enzyme:
(A-1) consists of an amino acid sequence set forth in SEQ ID NO: 23;
(A-2) consists of an amino acid sequence having a sequence identity of 90% or more with the amino acid sequence set forth in SEQ ID NO: 23, and has an enzymatic activity to N-acetylate a diamine compound to produce an N-acetyldiamine compound; or
(A-3) consists of an amino acid sequence in which 1 to 10 amino acids are deleted, substituted, inserted, and/or added in the amino acid sequence set forth in SEQ ID NO: 23, and has an enzymatic activity to N-acetylate a diamine compound to produce an N-acetyldiamine compound.

In a more preferred aspect, the N-acetylation enzyme (A-1) consists of an amino acid sequence set forth in SEQ ID NO: 23.

In another aspect, the N-acetylation enzyme is encoded by:
(B-1) DNA that consists of a base sequence set forth in SEQ ID NO: 24;
(B-2) DNA that hybridizes with DNA containing a base sequence complementary to the base sequence set forth in SEQ ID NO: 24 under stringent conditions, and encodes a protein having an enzymatic activity that N-acetylates a diamine compound to produce an N-acetyldiamine compound;
(B-3) DNA that consists of a base sequence having a sequence identity of 80% or more, 85% or more, 88% or more, 90% or more, 93% or more, 95% or more, 97% or more, 98% or more, or 99% or more with the base sequence set forth in SEQ ID NO: 24, and encodes a protein having an enzymatic activity that N-acetylates a diamine compound to produce an N-acetyldiamine compound;
(B-4) DNA that encodes a protein consisting of an amino acid sequence in which 1 to 10, 1 to 7, 1 to 5, or 1 to 3 amino acids are deleted, substituted, inserted, and/or added in an amino acid sequence of a protein encoded by the base sequence set forth in SEQ ID NO: 24, and encodes a protein having an enzymatic activity that N-acetylates a diamine compound to produce an N-acetyldiamine compound; or
(B-5) DNA that consists of a degenerate isomer of the base sequence set forth in SEQ ID NO: 24.

In a preferred aspect, the N-acetylation enzyme is encoded by:
(B-1) DNA that consists of a base sequence set forth in SEQ ID NO: 24;
(B-2) DNA that hybridizes with DNA containing a base sequence complementary to the base sequence set forth in SEQ ID NO: 24 under stringent conditions, and encodes a protein having an enzymatic activity that N-acetylates a diamine compound to produce an N-acetyldiamine compound;
(B-3) DNA that consists of a base sequence having a sequence identity of 90% or more with the base sequence set forth in SEQ ID NO: 24, and encodes a protein having an enzymatic activity that N-acetylates a diamine compound to produce an N-acetyldiamine compound;
(B-4) DNA that encodes a protein consisting of an amino acid sequence in which 1 to 10 amino acids are deleted, substituted, inserted, and/or added in an amino acid sequence of a protein encoded by the base sequence set forth in SEQ ID NO: 24, and encodes a protein having an enzymatic activity that N-acetylates a diamine compound to produce an N-acetyldiamine compound; or
(B-5) DNA that consists of a degenerate isomer of the base sequence set forth in SEQ ID NO: 24.

In the present specification, the "stringent condition" is, for example, a condition of about "1xSSC, 0.1%SDS, 60°C", a more severe condition of about "0.1xSSC, 0.1%SDS, 60°C", and a still more severe condition of about "0.1xSSC, 0.1%SDS, 68°C".

In a more preferred aspect, the N-acetylation enzyme is encoded by (B-1) DNA consisting of a base sequence set forth in SEQ ID NO: 24.

Note that, in the present specification, a proportion (%) of a "sequence identity" of a comparative amino acid sequence to a reference amino acid sequence is defined as a percentage of amino acid residues in the comparative sequence that are identical to the amino acid residues in the reference sequence when the sequences are aligned so that the identity between the two sequences is maximized, and if necessary, a gap is introduced into one or both of the two sequences. In this case, a conservative substitution is not considered as a part of the sequence identity. The sequence identity can be determined by using publicly available computer software, for example, using an alignment search tool such as Basic Local Alignment Search Tool (BLAST) (registered trademark, hereinafter, omitted). In an alignment, those skilled in the art can determine appropriate parameters to obtain the maximum alignment of the comparative sequence. The "sequence identity" of the nucleotide sequence can also be determined by a similar method.

The genetically modified microorganism obtained as described above is cultured and maintained under conditions suitable for its growth and/or maintenance in order to produce a diamine. A suitable medium composition, culture conditions, and culture time for transformants derived from various host microorganism cells can be easily selected by those skilled in the art.

Therefore, a second aspect of the present invention relates to a method for manufacturing a diamine compound, the manufacturing method including culturing the recombinant microorganism described above. Specifically, the manufacturing method includes a culture step of culturing the recombinant microorganism described above to obtain a culture of the recombinant microorganism and/or an extract of the culture.

The medium used in the culture step is as described in the invention A.

In a case where a raw material derived from biomass is used, the diamine as a product can be clearly distinguished from the synthetic raw material by the measurement method described in the invention A.

Regarding the medium, in a case where the host microorganism cannot assimilate the carbon sources such as cellulose and polysaccharides, the host microorganism can be adapted to produce a diamine using a carbon source by subjecting the host microorganism to the genetic engineering technique described in the invention A.

The culture form and the culture conditions are as described in the invention A.

The manufacturing method according to the present invention preferably further includes a mixing step of mixing the culture and/or the extract of the culture with a substrate compound to obtain a mixed solution.

In the culture and/or the mixed solution, as a result of the reaction, a diamine compound, which is a target compound, is produced. Therefore, in a more preferred aspect, the manufacturing method according to the present invention further includes a recovery step of recovering the diamine compound from the culture and/or the mixed solution.

The manufacturing method according to the present invention may include one or more steps selected from the steps described in the invention A.

As described above, the present invention is a halophilic and/or alkalophilic recombinant microorganism having a diamine producing ability, the recombinant microorganism containing one or more genetic modifications that suppress an N-acetylation enzyme that N-acetylates a diamine compound to produce an N-acetyldiamine compound. In the culture of the recombinant microorganism of the present invention, production of an N-acetyldiamine compound as a by-product can be suppressed. The recombinant microorganism of the present invention has a diamine producing ability, and can produce a diamine while suppressing production of a by-product. Therefore, the diamine as a target compound can be efficiently obtained. Furthermore, the recombinant microorganism according to the present invention is also expected to be applied to production of a diamine compound on an industrial scale.

In addition, in a case where the recombinant microorganism according to the present invention is alkalophilic, the microorganism is used for diamine production, such that there is no need to adjust the pH by adding an acid solution, and it is possible to avoid complication of the diamine production process. In addition, in a case where the recombinant microorganism according to the present invention is halophilic, the microorganism can grow even in a culture solution containing a salt, and the waste liquid containing a salt added in the separation step of separating the phase containing a diamine from the culture solution or the mixed solution (reaction solution) can be reused for culturing the microorganism according to the present invention, and thus, the wastewater treatment cost can be reduced.

Although the embodiments for implementing the present invention have been exemplified above, the embodiments described above are merely examples, and are not intended to limit the scope of the invention. The embodiments described above can be implemented in various other forms, and various omissions, substitutions, and changes can be made without departing from the gist of the invention.

### Examples

### <1> Invention A

Hereinafter, the present invention A will be described based on Examples, and the present invention A is not limited these Examples.

All the PCRs shown in the present Examples were performed using PrimeSTAR Max DNA Polymerase (trade name, manufactured by Takara Bio Inc.). In transformation of Bacillus pseudofirmus, Bacillus halodurans, and Bacillus marmarensis, an electroporation method was used. In the electroporation method, a cuvette having a width of 0.1 cm and containing 1 µl of plasmid DNA together with 60 µl of competent cells was attached to gene pulsar (manufactured by Bio-Rad Laboratories, Inc.), and a pulse of a voltage of 2.5 kV, a resistance of 200 Q, and a capacitance of 25 µF was applied to the cuvette. After reversion culture at 37°C for 3 hours, the cells were applied to 181 medium containing 10 ug/mL of chloramphenicol to obtain a transformant. The 181 medium composition is shown in Table A-1.

**[Table A-1]**

| Table A-1: 181 medium composition | |
|---|---|
| | [g/L] |
| Polypeptone | 5 |
| Yeast extract | 5 |
| K₂HPO₄ | 1 |
| MgSO₄·7H₂O | 0.2 |
| Glucose | 10 |
| Na₂CO₃ | 10 |

### Example A1: Construction of cadA gene expression plasmid for Bacillus pseudofirmus and acquisition of transformant

### (Example A1-a) Cloning of promoter region

Bacillus pseudofirmus OF4 strain (JCM 17055 strain, the present strain was provided by RIKEN BRC through National Resource Project of the Ministry of Education, Culture, Sports, Science and Technology) was cultured with shaking at 37°C in 181 medium (2 ml). After completion of the culture, bacterial cells were recovered from the culture solution, and a genomic DNA was extracted using Nucleo Spin Tissue (trade name, manufactured by MACHEREY-NAGEL). A promoter region (SEQ ID NO: 5) of the rpoD gene of Bacillus pseudofirmus was subjected to PCR amplification by a primer set having sequences set forth in SEQ ID NOs: 6 and 7 (fragment 1). The reaction conditions were set to 98°C (10 sec), 55°C (5 sec), 72°C (30 sec), and 30 cycles.

Plasmid pAL351 (deposited with National Institute of Technology and Evaluation, Patent Microorganisms Depositary (NPMD) (Address: Room 122, 2-5-8 Kazusa-Kamatari, Kisarazu, Chiba) on March 18, 2019, as NITE P-02918, and then was transferred to the international deposition under the provisions of the Budapest Treaty on July 28, 2020, and has been assigned Accession Number: NITE BP-02918) was subjected to PCR amplification by a primer set set forth in SEQ ID NO: 8 and SEQ ID NO: 9 (fragment 2). The reaction conditions were set to 98°C (10 sec), 55°C (5 sec), 72°C (30 sec), and 30 cycles. The fragment 2 was set as a vector side, and the fragment 1 was ligated as an insert to construct pALP01.

### (Example A1-b) Cloning of cadA Gene

Escherichia coli W3110 strain (NBRC 12713) was cultured with shaking in LB medium (2 mL) at 37°C. After completion of the culture, bacterial cells were recovered from the culture solution, and a genomic DNA was extracted using Nucleo Spin Tissue. PCR amplification was performed by a primer set having sequences set forth in SEQ ID NOs: 10 and 11 using the extracted genomic DNA as a template. The reaction conditions were set to 98°C (10 sec), 55°C (5 sec), 72°C (30 sec), and 30 cycles. The amplified fragment was ligated downstream of the promoter sequence of pALP01 to construct pAL328.

### (Example A1-c) Acquisition of transformant

pAL328 constructed in Example A1-b was transformed into each of:
· Bacillus pseudofirmus OF4 strain (JCM17055 strain, the strain was provided by RIKEN BRC through National Resource Project of the Ministry of Education, Culture, Sports, Science and Technology);
· Bacillus halodurans C-125 strain (JCM9153 strain, the strain was provided by RIKEN BRC through National Resource Project of the Ministry of Education, Culture, Sports, Science and Technology); and
· Bacillus marmarmarensis GMBE72 strain (JCM15719 strain, the strain was provided by RIKEN BRC through National Resource Project of the Ministry of Education, Culture, Sports, Science and Technology),
thereby obtaining
· AKRM-1 strain,
· AKRM-2 strain, and
· AKRM-3 strain, respectively.

On the other hand, as for a control transformation, plasmid pALP01 containing no cadA gene was transformed into each of:
· Bacillus pseudofirmus OF4 strain (JCM17055 strain, the strain was provided by RIKEN BRC through National Resource Project of the Ministry of Education, Culture, Sports, Science and Technology);
· Bacillus halodurans C-125 strain (JCM9153 strain, the strain was provided by RIKEN BRC through National Resource Project of the Ministry of Education, Culture, Sports, Science and Technology); and
· Bacillus marmarmarensis GMBE72 strain (JCM15719 strain, the strain was provided by RIKEN BRC through National Resource Project of the Ministry of Education, Culture, Sports, Science and Technology),
thereby obtaining
· AKRM-4 strain,
· AKRM-5 strain, and
· AKRM-6 strain, respectively.

Furthermore, as for the Bacillus pseudofirmus OF4 strain, a transformant containing a cadA gene expression cassette on a chromosome was also obtained. The present Bacillus pseudofirmus AKAL-001 strain was deposited with National Institute of Technology and Evaluation, Patent Microorganisms Depositary (NPMD) (Address: Room 122, 2-5-8 Kazusa-Kamatari, Kisarazu, Chiba) on March 18, 2019, as NITE P-02920, and then was transferred to the international deposition under the provisions of the Budapest Treaty on July 28, 2020, and has been assigned Accession Number: NITE BP-02920.

The sequences are shown below.
[Chem. 2]
(SEQ ID NO: 5) 
atacggcatc agactgatac accgttaaat aaaactcaag acacatggtt tctcctgaaa
aaggcaatca acttttgcaa acatagtgac ttgcggccta tatttatgta aaatatagta
gaaaggtaca cgaatacact tagtccttgt gtattgtgga tagagtaagc gtcttaaatt
taatgcgagg agattcataa
(SEQ ID NO: 6)
   atacggcatcagactgatacacc
(SEQ ID NO: 7)
   ttatgaatctcctcgcattaaatttaaga
(SEQ ID NO: 8)
   gtgcaaaatgcccaaaggct
(SEQ ID NO: 9)
   acgcgtccctctccttttgc
(SEQ ID NO: 10)
   atgaacgttattgcaatattgaatcacatg
(SEQ ID NO: 11)
   ttattttttgctttcttctttcaatacc

### Example A2: Cadaverine production by constructed strain (flask culture)

Each of the obtained transformants was cultured on a 181 medium plate at 37°C for 2 days to form colonies. 2 mL of 181 medium was placed in a 14 mL volumetric test tube, colonies were inoculated from the plate with a platinum loop and cultured at 37°C and 180 rpm until a sufficient turbidity was obtained, and the culture was used as a pre-culture solution for main culture.

Into a 150 mL volumetric Erlenmeyer flask, 30 mL of BS medium (composition is shown in Table A-2) was placed, and 0.3 mL of the pre-culture solution was added to perform main culture (cadaverine production test). The culture conditions were set to 37°C and 180 rpm. When the AKRM-1 to AKRM-6 strains were cultured, 10 mg/L of chloramphenicol was added to the medium for both the pre-culture and the main culture.

**[Table A-2]**

| Table A-2: BS medium composition | |
|---|---|
| | [g/L] |
| Na₂HPO₄ | 4.9 |
| (NH₄)₂SO₄ | 1 |
| K₂HPO₄ | 2 |
| Na₂SO₄ | 50 |
| MgSO₄·7H₂O | 0.2 |
| Yeast extract | 0.1 |
| CaCl₂·2H₂O | 0.0017 |
| FeSO₄·7H₂O | 0.0024 |
| ZnSO₄·7H₂O | 0.0003 |
| CoCl₂·6H₂O | 0.0024 |
| MnSO₄·7H₂O | 0.0024 |
| CuCl₂·2H₂O | 0.0002 |
| Na₂MoO₄ | 0.00025 |
| Na₂CO₃ | 10 |
| Casamino acid | 10 |
| Glucose | 20 |

The culture solution above was centrifuged at 10,000 g for 3 minutes to recover a supernatant, and a concentration of cadaverine in the culture supernatant was measured. Specifically, ion chromatography analysis (detector: electrical conductivity, column temperature: 30°C, flow rate: 0.35 mL/min, mobile phase: gradient of 8 mM methanesulfonic acid aqueous solution → 70 mM methanesulfonic acid aqueous solution) was performed by connecting CG-19 (guard column) and CS-19 (analytical column) (all are trade names, manufactured by Thermo Fisher Scientific Inc.) to quantify the concentration of cadaverine in the culture supernatant. Regarding the concentration of cadaverine, the results of comparison between the transformants of the present invention (AKRM-1 strain, AKRM-2 strain, AKRM-3 strain, and AKAL-001 strain) and the control strains (AKRM-4 strain, AKRM-5 strain, and AKRM-6 strain) are shown in Table A-3.

As compared to the control strains (AKRM-4 strain, AKRM-5 strain, and AKRM-6 strain), the transformants of the present invention (AKRM-1 strain, AKRM-2 strain, AKRM-3 strain, and AKAL-001 strain) produced more cadaverine with the lapse of culture time.

**[Table A-3]**

| Table A-3: Concentration (mg/L) of cadaverine produced in culture supernatant during flask culture | | | |
|---|---|---|---|
| | Culture time | | |
| | 24h | 48h | |
| AKRM-1 strain (Inventive Strain) | 180 | 261 | Plasmid |
| AKRM-2 strain (Inventive Strain) | 160 | 249 | Plasmid |
| AKRM-3 strain (Inventive Strain) | 170 | 180 | Plasmid |
| AKRM-4 strain (Control Strain) | 0 | 0 | |
| AKRM-5 strain (Control Strain) | 0 | 0 | |
| AKRM-6 strain (Control Strain) | 0 | 0 | |
| AKAL-001 strain (Inventive Strain) | 204 | 242 | Chromosome-inserted |

### Example A3: Cadaverine production by constructed strain (fermenter culture)

The AKRM-1 strain, the AKRM-4 strain, and the AKAL-001 strain were cultured on a 181 medium plate at 37°C for 2 days to form colonies. 100 mL of 181 medium was placed in a 500 mL volumetric Erlenmeyer flask, colonies were inoculated from the plate with a platinum loop and cultured at 37°C and 180 rpm until a sufficient turbidity was obtained, and the culture was used as a pre-culture solution for main culture.

In a 10 L volumetric jar culture device (model name: MDL-6 C, manufactured by Marubishi Bioengineering Co., Ltd.), BS jar medium containing 20 g/L of glucose (shown in Table A-4) was placed, and 100 mL of the pre-culture solution was added to perform main culture (cadaverine production test). When the AKRM-1 strain and the AKRM-4 strain were cultured, 10 mg/L of chloramphenicol was added to the medium for both the pre-culture and the main culture. The culture conditions were set to a culture temperature: 37°C, a culture pH: 7.5, alkali addition: 28% ammonia water, a stirring speed: 700 rpm, and an aeration speed: 0.1 vvm. In addition, a feed medium (composition is shown in Table A-5) was sequentially added during culture so that a concentration of glucose in the medium was 0 to 5 g/L. Sampling was performed over time during culture, and the concentration of cadaverine in the culture supernatant was quantified.

**[Table A-4]**

| Table A-4: BS jar medium composition | |
|---|---|
| | [g/L] |
| Na₂HPO₄ | 1 |
| K₂HPO₄ | 2 |
| MgSO₄·7H₂O | 1 |
| CaCl₂·2H₂O | 0.0034 |
| FeSO₄·7H₂O | 0.0048 |
| ZnSO₄·7H₂O | 0.0006 |
| CoCl₂·6H₂O | 0.0048 |
| MnSO₄·7H₂O | 0.0048 |
| CuCl₂·2H₂O | 0.004 |
| Na₂MoO₄ | 0.0005 |
| Na₂SO₄ | 50 |
| Yeast extract | 0.5 |
| (NH₄)₂SO₄ | 2 |
| Glucose | 20 |
| ADEKANOL (antifoaming agent) | 0.1 |

**[Table A-5]**

| Table A-5: Feed medium composition | |
|---|---|
| | [g/L] |
| Na₂HPO₄ | 1 |
| K₂HPO₄ | 2 |
| Na₂SO₄ | 50 |
| MgSO₄·7H₂O | 5 |
| MnSO₄·7H₂O | 0.1 |
| CaCl₂·2H₂O | 0.0034 |
| FeSO₄·7H₂O | 0.0048 |
| ZnSO₄·7H₂O | 0.0006 |
| CoCl₂·6H₂O | 0.0048 |
| CuCl₂·2H₂O | 0.0004 |
| Na₂MoO₄ | 0.00050 |
| Glucose | 680 |

The results are shown in Table A-6. The AKRM-4 strain did not produce cadaverine, the AKRM-1 strain expressing the cadA gene on the plasmid did not increase the concentration of cadaverine, and the AKAL-001 strain expressing the cadA gene on the chromosome increased the concentration of cadaverine with the lapse of culture time.

**[Table A-6]**

| | | | |
|---|---|---|---|
| Table A-6: Concentration (mg/L) of cadaverine produced in culture supernatant during fermenter culture | | | |

| | Culture time (h) | Cadaverine (g/L) | |
|---|---|---|---|
| AKRM-1 strain (Inventive Strain) | 67 | 0.24 | Plasmid |
| AKRM-4 strain (Control Strain) | 0 | 0 | |
| AKAL-001 strain (Inventive Strain) | 63 | 4.24 | Chromosome-inserted |

### Example A4: Cadaverine production with increased concentration of sodium sulfate (flask culture)

The AKAL-001 strain was cultured on a 181 medium plate at 37°C for 2 days to form colonies. 2 mL of 181 medium was placed in a 14 mL volumetric test tube, colonies were inoculated from the plate with a platinum loop and cultured at 37°C and 180 rpm until a sufficient turbidity was obtained, and the culture was used as a pre-culture solution for main culture.

In a 150 mL volumetric Erlenmeyer flask, 30 mL of two BS media (shown in Table A-2) were prepared. As shown in Table A-2, 50 g/L of sodium sulfate was added to one medium, and 100 g/L of sodium sulfate was added to the other medium. 0.3 mL of the pre-culture solution was added to perform main culture (cadaverine production test). The culture conditions were set to 37°C and 180 rpm.

The culture solution was centrifuged at 10,000 g for 3 minutes to recover a supernatant, and the results of measuring a concentration of cadaverine in the culture supernatant by the method described in Example A2 were shown in Table A-7. The AKAL-001 strain produced cadaverine even in the presence of sodium sulfate at a high concentration of 100 g/L, and showed an increase in concentration of cadaverine with the lapse of culture time.

**[Table A-7]**

| Table A-7: Concentration (mg/L) of cadaverine produced in culture supernatant during flask culture | | | |
|---|---|---|---|
| | Added Na₂SO₄ concentration | Culture time | |
| | | 17h | 46h |
| AKAL-001 strain (Inventive Strain) | 50 g/L | 241 | 245 |
| AKAL-001 strain (Inventive Strain) | 100 g/L | 199 | 230 |

### Example A5: Phase separability when salt compound at each concentration is added

Cadaverine (1,5-diaminopentane, manufactured by FUJIFILM Wako Pure Chemical Corporation), sodium chloride, sodium sulfate, and sodium carbonate were added at various concentrations to a simulated liquid for separation confirmation (composition is shown in Table A-8) to prepare 5 mL of each simulated liquid. After mixing with a vortex mixer, the mixture was allowed to stand at room temperature for 10 minutes, and the state of phase separation and the liquid amounts of the aqueous phase and the cadaverine phase were confirmed. In addition, a concentration of cadaverine contained in the aqueous phase was measured, the amount of cadaverine remaining in the aqueous phase was calculated, and a transfer rate from the aqueous phase to the cadaverine phase was calculated.

**[Table A-8]**

| Table A-8: Simulated liquid composition for separation confirmation | |
|---|---|
| | [g/L] |
| Na₂HPO₄ | 4.9 |
| (NH₄)₂SO₄ | 1 |
| K₂HPO₄ | 2 |
| MgSO₄·7H₂O | 0.2 |
| Yeast extract | 0.1 |
| CaCl₂·2H₂O | 0.0017 |
| FeSO₄·7H₂O | 0.0024 |
| ZnSO₄·7H₂O | 0.0003 |
| CoCl₂·6H₂O | 0.0024 |
| MnSO₄·7H₂O | 0.0024 |
| CuCl₂·2H₂O | 0.0002 |
| Na₂MoO₄ | 0.00025 |
| Casamino acid | 10 |
| Glucose | 20 |

The finally prepared solution composition and the separation results are shown in Table A-9. In the table, in the phase separation column, "×" indicates that phase separation does not occur, "O" indicates that the formed diamine phase is less than 10% of the total liquid amount in visual confirmation (for example, when the total liquid amount is 5 mL, the diamine phase is less than 0.5 mL), and "⊙" indicates that the formed diamine phase is generated in an amount of 10% or more of the total liquid amount in visual confirmation. The present evaluation criteria for phase separation also apply to the following tables. As a result of the test, it was confirmed that diamine phase separation occurred under the condition in which each inorganic salt was added at a high concentration.

**[Table A-9]**

| Table A-9: Phase separability when salt compound is added at each concentration | | | | | | | | |
|---|---|---|---|---|---|---|---|---|
| Cadaverine [g/L] | NaCl [g/L] | Na2SO4 \|g/L] | Na2CO3 [g/L] | Phase separation | Aqueous phase [mL] | Amine phase [mL] | Aqueous phase concentration [g/L] | Transfer rate [%] |
| 50 | 0 | | | × | 5.0 | | 48.1 | 0.0 |
| 50 | 120 | | | × | 5.0 | | | |
| 50 | 160 | | | × | 5.0 | | | |
| 50 | 200 | | | ○ | 4.9 | 0.1 | | |
| 50 | | 120 | | × | 5.0 | | | |
| 50 | | 160 | | ○ | 4.8 | 0.2 | | |
| 50 | | 200 | | ⊙ | 4.3 | 0.7 | 16.2 | 70.8 |
| 50 | | | 120 | × | 5.0 | | | |
| 50 | | | 160 | × | 5.0 | | | |
| 50 | | | 200 | ⊙ | 4.3 | 0. 7 | 20.7 | 62.7 |

### Example A6: Phase separability of cadaverine and hexamethylenediamine (HMDA)

Sodium sulfate and cadaverine (1,5-diaminopentane, manufactured by FUJIFILM Wako Pure Chemical Corporation) or hexamethylenediamine (manufactured by FUJIFILM Wako Pure Chemical Corporation) were added at various concentrations to a simulated liquid for separation confirmation (composition is shown in Table A-8) to prepare 5 mL of each simulated liquid. After mixing with a vortex mixer, the mixture was allowed to stand at room temperature for 10 minutes, and the state of phase separation and the liquid amounts of the aqueous phase and the cadaverine phase were confirmed. In addition, a concentration of cadaverine contained in the aqueous phase was measured, the amount of cadaverine remaining in the aqueous phase was calculated, and a transfer rate from the aqueous phase to the cadaverine phase was calculated.

The finally prepared solution composition and the separation results are shown in Table A-10. It was confirmed that both cadaverine and hexamethylenediamine (HMDA) were phase-separated in the presence of a high concentration of sodium sulfate.

**[Table A-10]**

| Table A-10: Phase separability of cadaverine and hexamethylenediamine | | | | | | | |
|---|---|---|---|---|---|---|---|
| Cadaverine [g/L] | HMDA [g/L] | Na2SO4 [g/L] | Phase separation | Aqueous phase [mL] | Amine phase [mL] | Aqueous phase concentration [g/L] | Transfer rate [%] |
| 100 | | | × | 5.0 | | | |
| 100 | | 100 | × | 5.0 | | | |
| 100 | | 150 | × | 5.0 | | | |
| 100 | | 200 | ⊙ | 3.3 | 1.7 | 30.0 | 89.8 |
| 150 | | | × | 5.0 | | | |
| 150 | | 100 | ⊙ | 0.1 | 4.9 | 53.5 | 65.0 |
| 150 | | 150 | ⊙ | 2.5 | 2.5 | 25.6 | 91.5 |
| 150 | | 200 | ⊙ | 2.7 | 2.3 | 15.0 | 95.4 |
| | 100 | | × | 5.0 | | | |
| | 100 | 100 | × | 5.0 | | | |
| | 100 | 150 | × | 5.0 | | | |
| | 100 | 200 | ⊙ | 3.5 | 1.5 | 31.1 | 90.7 |
| | 160 | | × | 5.0 | | | |
| | 160 | 100 | ⊙ | 1.8 | 3.2 | 55.6 | 77.7 |
| | 160 | 150 | ⊙ | 2.5 | 2.5 | 23.3 | 92.7 |
| | 160 | 200 | ⊙ | 2.8 | 2.2 | 11.9 | 96.7 |

### Example A7: Solvent extractability when sodium carbonate is added at each concentration (cadaverine)

Sodium carbonate and cadaverine (1,5-diaminopentane, manufactured by FUJIFILM Wako Pure Chemical Corporation) were added at each concentration to a simulated liquid for separation confirmation (composition is shown in Table A-8) to prepare 5 mL of each simulated liquid. An equivalent amount (5 mL) of n-butanol was added to the simulated liquid, mixing was performed with a vortex mixer, and then the mixture was allowed to stand at room temperature for 10 minutes. A concentration of cadaverine contained in the aqueous phase was measured, the amount of cadaverine remaining in the aqueous phase was calculated, and a transfer rate from the aqueous phase to the solvent phase was calculated.

The finally prepared solution composition and the separation results are shown in Table A-11. It was confirmed that the extraction efficiency into the solvent was improved as the concentration of sodium carbonate increased.

**[Table A-11]**

| Table A-11: Solvent extractability when sodium carbonate at each concentration is added (cadaverine) | | | | |
|---|---|---|---|---|
| Cadaverine [g/L] | Na2CO3 [g/L] | Extraction solvent | Aqueous phase concentration [g/L] | Transfer rate [%] |
| 78.3 | 0 | n-Butanol | 37.3 | 69.6 |
| 78.3 | 20 | | 31.1 | 72.5 |
| 78.3 | 100 | | 13.6 | 87.4 |
| 78.3 | 140 | | 8.9 | 91.0 |
| 78.3 | 180 | | 6.8 | 94.3 |
| 78.3 | 220 | | 5.3 | 95.7 |

### Example A8: Solvent extractability when sodium sulfate is added at each concentration (hexamethylenediamine)

Sodium sulfate and hexamethylenediamine were added at each concentration to a simulated liquid for separation confirmation (composition is shown in Table A-8) to prepare 5 mL of each simulated liquid. An equivalent amount (5 mL) of hexane or 2-ethyl-1-hexanol was added to the simulated liquid, mixing was performed with a vortex mixer, and then the mixture was allowed to stand at room temperature for 10 minutes. A concentration of hexamethylenediamine contained in the solvent phase was measured, and a transfer rate from the aqueous phase to the solvent phase was calculated.

The finally prepared solution composition and the separation results are shown in Table A-12. It was confirmed that the extraction efficiency into 2-ethyl-1-hexanol was improved and the extraction efficiency into n-hexane was significantly low as the concentration of sodium sulfate increased.

**[Table A-12]**

| Table A-12: Solvent extractability when sodium sulfate at each concentration is added (hexamethylenediamine) | | | | |
|---|---|---|---|---|
| HMDA [g/L] | Na2CO4 [g/L] | Extraction solvent | Solvent phase concentration [g/L] | Transfer rate [%] |
| 100 | 0 | n-Hexane | 10 mg/L or less | |
| 100 | 100 | | 10 mg/L or less | |
| 100 | 200 | | 10 mg/L or less | |
| 100 | 0 | 2-Ethyl-1-hexanol | 27.0 | 27.0 |
| 100 | 100 | | 42.9 | 42.9 |
| 100 | 200 | | 56.7 | 56.7 |

### Example A9: Phase separability in diamine-added culture solution

30 mL of the culture supernatant of the flask culture described in Example A4 was filtered through a 0.22 µm filter, cadaverine (1,5-diaminopentane, manufactured by FUJIFILM Wako Pure Chemical Corporation) or hexamethylenediamine (manufactured by FUJIFILM Wako Pure Chemical Corporation) was added thereto so as to have each concentration, and the mixture was concentrated to 2 times by an evaporator (yield: 15 mL). After mixing the present concentrate with a vortex mixer, the mixture was allowed to stand at room temperature for 10 minutes, and the state of phase separation and the liquid amounts of the aqueous phase and the cadaverine phase were confirmed. In addition, a concentration of cadaverine contained in the aqueous phase was measured, the amount of cadaverine remaining in the aqueous phase was calculated, and a transfer rate from the aqueous phase to the cadaverine phase was calculated. The finally prepared solution composition and the separation results are shown in Table A-13. It was confirmed that both cadaverine and hexamethylenediamine were phase-separated when a high concentration of sodium sulfate was present in the culture solution.

**[Table A-13]**

| Table A-13: Phase separability in diamine added culture solution | | | | | | | |
|---|---|---|---|---|---|---|---|
| Culture solution | Cadaverine [g/L] | HMDA [g/L] | Phase separation | Aqueous phase [mL] | Amine phase [mL] | Aqueous phase concentration [g/L] | Transfer rate [%] |
| Table A-7 50 g/L 2-fold concentration | 100 | | × | 5.0 | | | |
| | 150 | | × | 5.0 | | | |
| | | 100 | × | 5.0 | | | |
| | | 150 | ⊙ | 2.0 | 3.0 | 50.0 | 86.7 |
| Table A-7 100 g/L 2-fold concentration | 100 | | ⊙ | 3.5 | 1.5 | 32.2 | 77.5 |
| | 150 | | ⊙ | 2.8 | 2.2 | 17.3 | 93.5 |
| | | 100 | ⊙ | 3.6 | 1.4 | 33.1 | 76.2 |
| | | 150 | ⊙ | 2.8 | 2.2 | 17.0 | 93.7 |

### Example A10: Culture in which aqueous phase after phase separation is reused

100 mL of a fermenter culture solution (culture for 63 hours) of the AKAL-001 strain described in Example A3 was recovered, and centrifugation was performed to recover a supernatant. 100 mL of the culture supernatant was filtered through a 0.22 um filter and concentrated to 2 times with an evaporator (yield: 50 mL). Hexamethylenediamine (manufactured by FUJIFILM Wako Pure Chemical Corporation) was added to the present concentrate so as to have a final concentration of 150 g/L. After mixing with a vortex mixer, the mixture was allowed to stand at room temperature for 10 minutes, and then the formation of the phase separation was confirmed. The aqueous phase was 20 mL, and the concentration of hexamethylenediamine was 48.5 g/L. The aqueous phase was recovered with a pipette, and the pH was adjusted to 7.5 with sulfuric acid, 30 mL of distilled water was added, and then the volume was adjusted to 50 mL. The present solution and a new BS medium (composition is shown in Table A-14) were mixed at various ratios to prepare a mixture in a 150 mL volumetric Erlenmeyer flask.

**[Table A-14]**

| Table A-14: Simulated liquid composition for separation confirmation | |
|---|---|
| | [g/L] |
| Na₂HPO₄ | 4.9 |
| (NH₄)₂SO₄ | 1 |
| K₂HPO₄ | 2 |
| Na₂SO₄ | 50 |
| MgSO₄·7H₂O | 0.2 |
| Yeast extract | 0.1 |
| CaCl₂·2H₂O | 0.0017 |
| FeSO₄·7H₂O | 0.0024 |
| ZnSO₄·7H₂O | 0.0003 |
| CoCl₂·6H₂O | 0.0024 |
| MnSO₄·7H₂O | 0.0024 |
| CuCl₂·2H₂O | 0.0002 |
| Na₂MoO₄ | 0.0025 |
| Glucose | 20 |

Bacillus pseudofirmus OF4 strain (JCM 17055 strain, the present strain was provided by RIKEN BRC through National Resource Project of the Ministry of Education, Culture, Sports, Science and Technology) was cultured on a 181 medium plate at 37°C for 2 days to form colonies. 2 mL of 181 medium was placed in a 14 mL volumetric test tube, colonies were inoculated from the plate with a platinum loop and cultured at 37°C and 180 rpm until a sufficient turbidity was obtained, and the culture was used as a pre-culture solution for main culture.

0.1 mL of the pre-culture solution was added to the medium prepared in the flask to perform main culture. The culture conditions were set to 37°C and 180 rpm. After 49 hours of culture, the culture solution was sampled, and a turbidity (OD600) measurement was performed.

The results are shown in Table A-15. It was confirmed that the aqueous phase after the phase separation can be reused as a medium.

**[Table A-15]**

| Table A-15: Culture reusing aqueous phase after phase separation | | |
|---|---|---|
| Medium [mL] | Aqueous phase after phase separation [mL] | OD660 after 49 h of culture |
| 10 | 0 | 0.88 |
| 8 | 2 | 0.83 |
| 5 | 5 | 0. 94 |
| 2 | 8 | 0.84 |
| 0 | 10 | 0.36 |

As shown the results described in the above Examples, diamines could be produced using a microorganism having both halophilicity and alkalophilicity as a host. In addition, phase-separation of diamines was confirmed in the presence of sodium carbonate or sodium sulfate at a high salt concentration, and the extraction efficiency at the time of solvent extraction was improved.

By concentrating the culture supernatant containing a diamine, it can be expected that the concentrations of the diamine and the inorganic salt increase and the separation efficiency is further improved. In addition, since the recombinant microorganism according to the present invention is used, the pH adjustment step by adding an acid solution can be omitted. Furthermore, water containing a high concentration of a salt generated when the diamine is isolated can be reused, and the wastewater treatment cost can be reduced.

### <2> Invention B

Hereinafter, the present invention B will be described based on Examples, and the present invention B is not limited these Examples.

All the PCRs shown in the present Examples were performed using PrimeSTAR Max DNA Polymerase (trade name, manufactured by Takara Bio Inc.). In transformation of Bacillus pseudofirmus, an electroporation method was used. In the electroporation method, a cuvette having a width of 0.1 cm and containing 1 µl of plasmid DNA together with 60 µl of a competent cell was attached to a gene pulsar (manufactured by Bio-Rad), and a pulse having a voltage of 2.5 kV, a resistance of 200 Ω, and a capacitance of 25 µF was applied to the cuvette. After reversion culture at 30°C for 3 hours, the cells were applied to 181 medium containing 10 ug/mL of chloramphenicol to obtain a transformant. The 181 medium composition is shown in Table B-1.

**[Table B-1]**

| Table B-1: 181 medium composition | |
|---|---|
| | [g/L] |
| Polypeptone | 5 |
| Yeast extract | 5 |
| K₂HPO₄ | 1 |
| MgSO₄·7H₂O | 0.2 |
| Glucose | 10 |
| Na₂CO₃ | 10 |

### Example B1: Acquisition of gene-disrupted strain

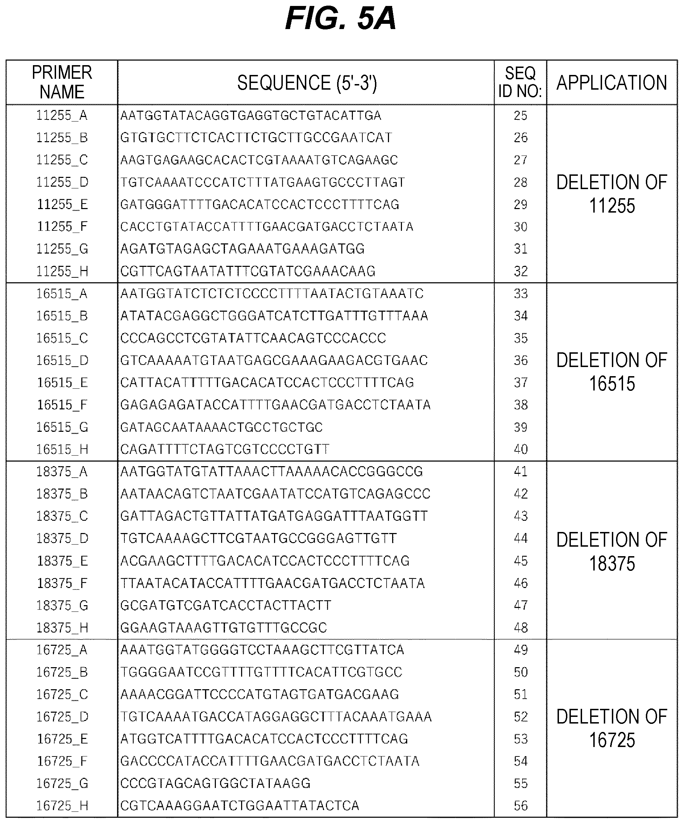

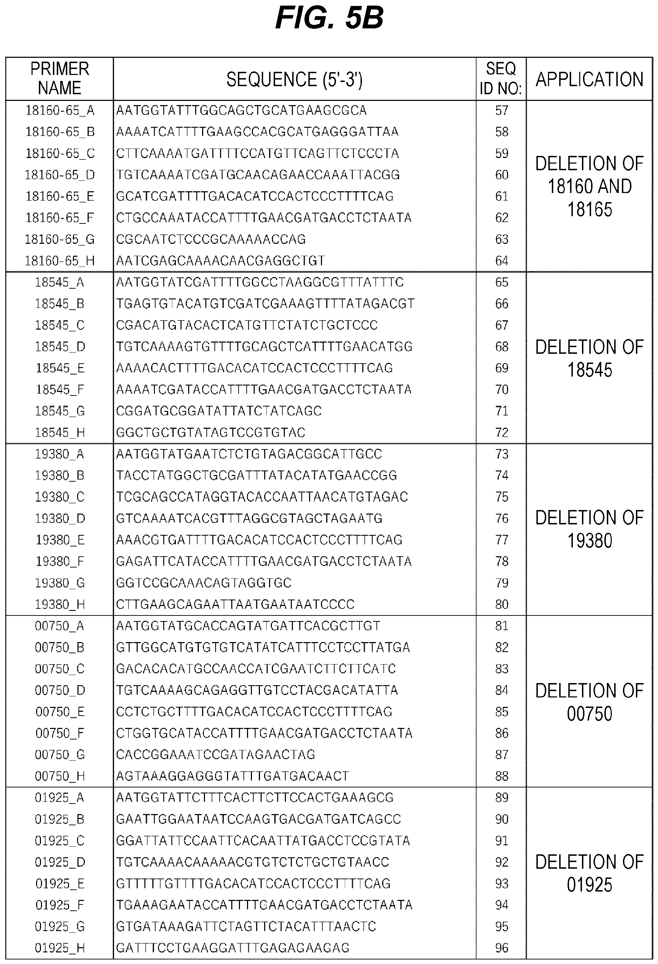

From the annotation information of Bacillus pseudofirmus OF4 strain published on the NCBI database, 39 genes encoding acetyltransferases were selected as candidate genes encoding an N-acetylation enzyme of a diamine. Among the 39 candidates,
gene-disrupted strains of:
   · BpOF4_11255 gene (GenBank: ADC50304);
   · BpOF4_16515 gene (GenBank: ADC51348);
   · BpOF4_18375 gene (GenBank: ADC51716);
   · BpOF4_16725 gene (GenBank: ADC51388);
   · BpOF4_18160-65 gene (GenBank: ADC51673-4);
   · BpOF4_18545 gene (GenBank: ADC51750);
   · BpOF4_19380 gene (GenBank: ADC51915);
   · BpOF4_00750 gene (GenBank: ADC48219); and
   · BpOF4_01925 gene (GenBank: ADC48452)
were prepared by a genetic manipulation into chromosomes by homologous recombination. The primer sequences used are illustrated in Figs. 5A and 5B.

### (Example B1-a) Preparation of gene-disrupted plasmid

Bacillus pseudofirmus OF4 strain (JCM 17055 strain, the present strain was provided by RIKEN BRC through National Resource Project of the Ministry of Education, Culture, Sports, Science and Technology) was cultured with shaking at 37°C in 181 medium (2 ml). After completion of the culture, bacterial cells were recovered from the culture solution, and a genomic DNA was extracted using Nucleo Spin Tissue (trade name, manufactured by MACHEREY-NAGEL) . The homology region was subjected to PCR amplification using a primer set in which "A" and "B" were added to the end of the primer name and a primer set in which "C" and "D" were added to the end of the primer name (see Fig. 5) to obtain fragments 1 and 2. The reaction conditions were set to 98°C (10 sec), 55°C (5 sec), 72°C (30 sec), and 30 cycles.

Plasmid pAL351 (deposited with National Institute of Technology and Evaluation, Patent Microorganisms Depositary (NPMD) on March 18, 2019, Accession Number: NITE BP-02918) was subjected to PCR amplification using a primer set in which "E" and "F" were added to the end of the primer name (see Fig. 5) to obtain a fragment 3. The reaction conditions were set to 98°C (10 sec), 55°C (5 sec), 72°C (30 sec), and 30 cycles. The fragment 3 was used as a vector side, and the fragments 1 and 2 were ligated as an insert to construct pAKNU01 to 09.

### (Example B1-b) Acquisition of transformant

pAKNU01 to pAKNU09 constructed in Example B1-a were transformed into Bacillus pseudofirmus AKAL-001 strain (deposited with National Institute of Technology and Evaluation, Patent Microorganisms Depositary (NPMD), Accession Number: NITE BP-02920) to obtain AKALp-101 strain to AKALp-109 strain.

### (Example B1-c) Acquisition of chromosome-inserted strain

The AKALp-101 strain to the AKALp-109 strain obtained in Example B1-b were cultured at 30°C in 181 medium containing 10 ug/mL of chloramphenicol for 1 day, diluted to 100 times, and applied to 181 medium containing 10 µg/mL of chloramphenicol, and culture was performed at 43°C, thereby obtaining chromosome-inserted strains AKALp-201 to AKALp-209 in which the plasmid was subjected to homologous recombination into the chromosome.

### (Example B1-d) Acquisition of yjbC gene-disrupted strain

The AKALp-201 to AKALp-209 strains obtained in Example B1-c were cultured at 30°C in 181 medium containing 10 ug/mL of chloramphenicol for 1 day, diluted to 100 times, and applied to 181 medium containing 5 mM 4-chlorophenylalanine, and culture was performed at 30°C, thereby obtaining gene-disrupted strains AKDNC-001 to AKDNC-009 in which the plasmid and each gene region were removed from the chromosome. The deletion of the gene was confirmed by PCR using a primer set in which "G" and "H" were added to the end of the primer name (see Fig. 5). The reaction conditions were set to 98°C (10 sec), 55°C (5 sec), 72°C (30 sec), and 30 cycles.

The correspondence between the constructed strain and the gene is shown in the following table.

| [Table B-2] | | |
|---|---|---|
| Strain | Gene | GenBank |
| AKDNC-001 strain | BpOF4_11255 | ADC50304 |
| AKDNC-002 strain | BpOF4_16515 | ADC51348 |
| AKDNC-003 strain | BpOF4_18375 | ADC51716 |
| AKDNC-004 strain | BpOF4_16725 | ADC51388 |
| AKDNC-005 strain | BpOF4_18160-65 | ADC51673-4 |
| AKDNC-006 strain | BpOF4_18545 | ADC51750 |
| AKDNC-007 strain | BpOF4_19380 | ADC51915 |
| AKDNC-008 strain | BpOF4_00750 | ADC48219 |
| AKDNC-009 strain | BpOF4_01925 | ADC48452 |

### Example B2: Evaluation of N-acetylcadaverine producing ability in constructed strain (fermenter culture)

The AKAL-001 strain and the AKDNC-001 to 009 strains were cultured on a 181 medium plate at 37°C for 1 day to form colonies. 2 mL of 181 medium was placed in a 14 mL volumetric test tube, colonies were inoculated from the plate with a platinum loop and cultured at 37°C and 180 rpm until a sufficient turbidity was obtained, and the culture was used as a pre-culture solution for main culture.

In a 100 mL volumetric jar culture device (model name: Bio Jr.8, manufactured by Biott Corporation), BS jar medium containing 30 g/L of glucose (shown in Table B-3) was placed, and 1 mL of the pre-culture solution was added to perform main culture (cadaverine production test). The culture conditions were set to a culture temperature: 37°C, a culture pH: 7.5, alkali addition: 10% ammonia water, a stirring speed: 750 rpm, and an aeration speed: 0.1 vvm. Sampling was performed over time during culture, and a bacterial cell turbidity in the culture solution and a concentration of a diamine in the culture supernatant were quantified. After 24 hours from the start of the culture, 6 ml of a 50% glucose solution was added.

The culture solution was centrifuged at 10,000 g for 3 minutes to recover a supernatant, and a concentration of cadaverine and a concentration of N-acetylcadaverine in the culture supernatant were measured. Specifically, ion chromatography analysis (detector: electrical conductivity, column temperature: 30°C, flow rate: 0.35 mL/min, mobile phase: gradient of 8 mM methanesulfonic acid aqueous solution → 70 mM methanesulfonic acid aqueous solution) was performed by connecting CG-19 (guard column) and CS-19 (analytical column) (all are trade names, manufactured by Thermo Fisher Scientific Inc.) to quantify the concentration of cadaverine and the concentration of N-acetylcadaverine in the culture supernatant.

**[Table B-3]**

| Table B-3: BS jar medium composition | |
|---|---|
| | [g/L] |
| Na₂HPO₄ | 1 |
| (NH₄)₂SO₄ | 4 |
| Na₂SO₄ | 50 |
| K₂HPO₄ | 2 |
| MgSO₄·7H₂O | 1 |
| Yeast extract | 0.5 |
| CaCl₂·2H₂O | 0.0034 |
| FeSO₄·7H₂O | 0.0048 |
| ZnSO₄·7H₂O | 0.0006 |
| CoCl₂·6H₂O | 0.0048 |
| MnSO₄·7H₂O | 0.0048 |
| CuCl₂·2H₂O | 0.0004 |
| Na₂MoO₄ | 0.0005 |
| Calcium pantothenate | 0.03 |
| Folic acid | 0.01 |
| myo-inositol | 0.01 |
| Nicotinic acid amide | 0.009 |
| p-Aminobenzoic acid | 0.01 |
| Pyridoxine hydrochloride | 0.01 |
| Riboflavin | 0.01 |
| Thiamine hydrochloride | 0.0075 |
| Biotin | 0.0045 |
| Citric acid 3Na·2H₂O | 0.0003 |
| Glucose | 30 |
| Theophylline | 0.09 |
| ADEKANOL (antifoaming agent) | 0.1 |

A bacterial cell density (OD) and concentrations (g/l) of cadaverine and N-acetylcadaverine after 40 hours of the culture are shown in Table B-4. Cadaverine and N-acetylcadaverine were detected from the culture supernatants of the AKAL-043 strain and the AKDNC-001 to 008 strains. On the other hand, cadaverine was detected but N-acetylcadaverine was not detected from the culture supernatant of the AKDNC-009 strain (Table B-4). The AKDNC-009 strain had a 14% increase in concentration of cadaverine compared to the AKAL-043 strain.

**[Table B-4]**

| Strain | OD | Cadaverine (g/l) | Acetylcadaverine (g/l) |
|---|---|---|---|
| AKAL-043 strain | 15.8 | 3.90 | 1.30 |
| AKDNC-001 strain | 17.8 | 4.42 | 1.20 |
| AKDNC-002 strain | 17.6 | 4.47 | 1.24 |
| AKDNC-003 strain | 24.2 | 4.72 | 1.65 |
| AKDNC-004 strain | 18.3 | 4.29 | 1.16 |
| AKDNC-005 strain | 14.8 | 3.36 | 0.86 |
| AKDNC-006 strain | 16.3 | 3.23 | 1.01 |
| AKDNC-007 strain | 15.8 | 3.90 | 1.30 |
| AKDNC-008 strain | 28.3 | 1.73 | 1.60 |
| AKDNC-009 strain | 27.3 | 4.44 | 0.00 |

From the results in Table B-4, it is understood that the strain in which the production amount of acetylcadaverine is reduced by 0.5 g/l or more as compared with the AKAL-001 strain which is a non-mutant strain has a lower activity to produce an N-acetyldiamine compound than the non-mutant strain as compared with the wild-type strain.

### Industrial Applicability

The present invention can be used for industrial fermentation production of a diamine. Since the present invention can also suppress production of a by-product and efficiently produce a diamine, it is expected that the fermentation of the recombinant microorganism of the present invention can be used for the production of a diamine on an industrial scale.

## Claims

1. A halophilic and/or alkalophilic recombinant microorganism having a diamine producing ability, wherein
the diamine is represented by formula NH₂CH₂(CH₂)ₙCH₂NH₂ (in the formula, n is an integer of 0 to 10), and
the recombinant microorganism resulting from modification of a halophilic and/or alkalophilic host microorganism so as to impart a diamine producing ability.

2. The recombinant microorganism according to claim 1, wherein n in the formula is 3 or 4.

3. The recombinant microorganism according to claim 2, wherein the host microorganism includes one or more genetic manipulations to induce overproduction of L-lysine decarboxylase (EC 4.1.1.18).

4. The recombinant microorganism according to claim 3, wherein the genetic manipulation is one or more manipulations selected from the group consisting of the following (A), (B), (C), (D), and (E):
(A) a manipulation of introducing an exogenous gene encoding the L-lysine decarboxylase into the host microorganism;
(B) a manipulation of increasing a copy number of an endogenous gene of the L-lysine decarboxylase in the host microorganism;
(C) a manipulation of introducing a mutation into an expression regulatory region of the endogenous gene of the L-lysine decarboxylase in the host microorganism;
(D) a manipulation of replacing the expression regulatory region of the endogenous gene of the L-lysine decarboxylase in the host microorganism by an exogenous regulatory region which enables high expression; and
(E) a manipulation of deleting the regulatory region of the endogenous gene of the L-lysine decarboxylase in the host microorganism.

5. The recombinant microorganism according to any one of claims 2 to 4, wherein the recombinant microorganism contains:
a base sequence having a homology of 85% or more, 90% or more, 92% or more, 95% or more, 98% or more, or 99% or more with a base sequence set forth in SEQ ID NO: 2 or SEQ ID NO: 4, or
a base sequence having a homology of 85% or more, 90% or more, 92% or more, 95% or more, 98% or more, or 99% or more with a base sequence encoding an amino acid sequence set forth in SEQ ID NO: 1 or 3.

6. The recombinant microorganism according to claim 2 or 3, wherein the recombinant microorganism is further modified by a mutation manipulation or a genetic recombination manipulation for improving a lysine producing ability.

7. The recombinant microorganism according to claim 6, wherein the mutation manipulation or the genetic recombination manipulation is a manipulation of canceling a feedback inhibition on at least one of aspartokinase III (EC 2.7.2.4) and 4-hydroxy-tetrahydrodipicolinate synthase (EC 4.3.3.7).

8. The recombinant microorganism according to claim 1 or 2, wherein the host microorganism is selected from the group consisting of Bacillus pseudofirmus, Bacillus halodurans, and Bacillus marmarensis.

9. The recombinant microorganism according to claim 1 or 2, wherein the host microorganism is Bacillus pseudofirmus.

10. A method for manufacturing a diamine represented by formula NH₂CH₂(CH₂)ₙCH₂NH₂ (in the formula, n is an integer of 0 to 10) using the recombinant microorganism according to claim 1 or 2.

11. The manufacturing method according to claim 10, wherein n in the formula is 3 or 4.

12. The manufacturing method according to claim 10, the method comprising a culture step of culturing the recombinant microorganism in a medium containing 5 mass% or more of an inorganic salt to obtain a culture solution containing a diamine.

13. The manufacturing method according to claim 10, the method comprises:
a culture step of culturing the recombinant microorganism to obtain a culture solution containing bacterial cells; and
a reaction step of bringing the culture solution and/or the bacterial cells into contact with an aqueous solution containing 5 mass% or more of an inorganic salt and lysine to obtain a reaction solution containing 1,5-pentanediamine.

14. The manufacturing method according to claim 12, the method comprising a removal step of removing the recombinant microorganism from the culture solution or the reaction solution.

15. The manufacturing method according to claim 14, the method comprising a concentration step of concentrating the culture solution or the reaction solution.

16. The manufacturing method according to claim 12, the method comprising a pH adjustment step of adjusting a pH of the culture solution or the reaction solution to 12 or higher.

17. The manufacturing method according to claim 12, the method comprising a separation step of performing a phase separation into a phase containing a diamine and an aqueous phase containing the inorganic salt from the culture solution or the reaction solution.

18. The manufacturing method according to claim 12, wherein in the separation step, a phase separation into a phase containing a diamine and an organic solvent and an aqueous phase is performed by adding an organic solvent to the culture solution or the reaction solution.

19. The manufacturing method according to claim 17, wherein in the separation step, an alkali compound is not added.

20. The manufacturing method according to claim 12, wherein the inorganic salt is sodium carbonate and/or sodium sulfate.

21. The manufacturing method according to claim 12, wherein
the diamine produced by the culture step and/or the reaction step is in one or more forms selected from the group consisting of a carbonate salt, a bicarbonate salt, a bisbicarbonate salt, a carbamate salt, and a biscarbamate salt, and
in the concentration step, the diamine in the forms of the salts is converted into a free base and carbon dioxide to separate the carbon dioxide.

22. The manufacturing method according to claim 18, wherein the organic solvent is one or more selected from the group consisting of hexane, butanol, and 2-ethyl-1-hexanol.

23. The manufacturing method according to claim 12, wherein the diamine is produced by fermentation of at least one or more of sugar, carbon dioxide, synthetic gas, methanol, and amino acid by the recombinant microorganism.

24. The manufacturing method according to claim 17, the method comprising reusing the separated aqueous phase containing the inorganic salt as a culture solution.

25. The manufacturing method according to claim 12, the method comprising a purification step of purifying the obtained diamine.

26. A halophilic and/or alkalophilic recombinant microorganism having a diamine producing ability, the recombinant microorganism comprising one or more genetic modifications that suppress an N-acetylation enzyme that N-acetylates a diamine compound to produce an N-acetyldiamine compound.

27. The recombinant microorganism according to claim 26, wherein the genetic modification is:
• a modification that suppresses expression of an endogenous gene encoding the N-acetylation enzyme; or
• a modification that reduces an activity of the N-acetylation enzyme.

28. The recombinant microorganism according to claim 26 or 27, wherein an N-acetyldiamine compound producing ability is suppressed or eliminated as compared with the producing ability of a non-mutant strain that does not contain the genetic modification.

29. The recombinant microorganism according to claim 26 or 27, wherein a gene encoding the N-acetylation enzyme is yjbC gene.

30. The recombinant microorganism according to claim 26 or 27, wherein
the N-acetylation enzyme:
(A) (A-1) consists of an amino acid sequence set forth in SEQ ID NO: 23;
(A-2) consists of an amino acid sequence having a sequence identity of 85% or more, 90% or more, 95% or more, 97% or more, 98% or more, or 99% or more with the amino acid sequence set forth in SEQ ID NO: 23, and has an enzymatic activity to N-acetylate a diamine compound to produce an N-acetyldiamine compound; or
(A-3) consists of an amino acid sequence in which 1 to 10, 1 to 7, 1 to 5, or 1 to 3 amino acids are deleted, substituted, inserted, and/or added in the amino acid sequence set forth in SEQ ID NO: 23, and has an enzymatic activity to N-acetylate a diamine compound to produce an N-acetyldiamine compound, or
the N-acetylation enzyme is encoded by:
(B) (B-1) DNA that consists of a base sequence set forth in SEQ ID NO: 24;
(B-2) DNA that hybridizes with DNA containing a base sequence complementary to the base sequence set forth in SEQ ID NO: 24 under stringent conditions, and encodes a protein having an enzymatic activity that N-acetylates a diamine compound to produce an N-acetyldiamine compound;
(B-3) DNA that consists of a base sequence having a sequence identity of 85% or more, 90% or more, 95% or more, 97% or more, 98% or more, or 99% or more with the base sequence set forth in SEQ ID NO: 24, and encodes a protein having an enzymatic activity that N-acetylates a diamine compound to produce an N-acetyldiamine compound;
(B-4) DNA that encodes a protein consisting of an amino acid sequence in which 1 to 10, 1 to 7, 1 to 5, or 1 to 3 amino acids are deleted, substituted, inserted, and/or added in an amino acid sequence of a protein encoded by the base sequence set forth in SEQ ID NO: 24, and encodes a protein having an enzymatic activity that N-acetylates a diamine compound to produce an N-acetyldiamine compound; or
(B-5) DNA that consists of a degenerate isomer of the base sequence set forth in SEQ ID NO: 24.

31. The recombinant microorganism according to claim 26 or 27, the recombinant microorganism resulting from one or more genetic modifications of a halophilic and/or alkalophilic host microorganism to suppress the N-acetylation enzyme.

32. The recombinant microorganism according to claim 26 or 27, wherein the host microorganism is selected from the group consisting of Bacillus pseudofirmus, Bacillus halodurans, and Bacillus marmarensis.

33. The recombinant microorganism according to claim 26 or 27, wherein the diamine compound is represented by formula NH₂CH₂(CH₂)ₙCH₂NH₂ (in the formula, n is 0, 1, 2, 3, 4, 5, 6, 7, 8, 9, or 10).

34. The recombinant microorganism according to claim 26 or 27, wherein the diamine compound is cadaverine.

35. A method for manufacturing a diamine compound, the method comprising a culture step of culturing the recombinant microorganism according to claim 26 or 27 to obtain a culture of the recombinant microorganism and/or an extract of the culture.

36. The manufacturing method according to claim 35, the method further comprising a mixing step of mixing the culture and/or the extract of the culture with a substrate compound to obtain a mixed solution.

37. The manufacturing method according to claim 35, the method further comprising a recovery step of recovering a diamine compound from the culture or the mixed solution.
